# EUROPEAN PATENT APPLICATION

(11) **EP 4 748 818 A1**
(43) Date of publication of application: **27.05.2026**
(21) Application number: 25207676.5
(22) Date of filing: 09.10.2025
(51) Int. Cl.: C07C 29/00, C07C 29/132, C07C 29/60, C07C 45/52, C07C 51/00, C01B 3/22

(54) **METHOD FOR DECOMPOSING POLYOL OR POLYOL ESTER**

(30) Priority: 22.11.2024 JP 2024204253
(71) Applicant: AC Biode Ltd., Kyoto-shi, Kyoto 606-0024 (JP)
(72) Inventor: MIZUSAWA, Atsushi, Kyoto, 619-0237 (JP); VANJARAPU, Kesava Rao, Kyoto, 619-0237 (JP); RAHMAN, Habibur, Kyoto, 619-0237 (JP); KUNZMANN, Robert, Kyoto, 619-0237 (JP); KUBO, Tadashi, Kyoto, 619-0237 (JP)
(74) Representative: Hoeger, Stellrecht & Partner Patentanwälte mbB

(57) **Abstract**

In a method for decomposing a polyol or a polyol ester according to the present invention, a substance to be decomposed containing at least one of a polyol and a polyol ester is brought into contact with a solid of a metal oxide or a metal hydroxide having a standard oxidation-reduction potential different from that of water in predetermined reaction water.

## Description

### TECHNICAL FIELD

The present invention relates to a method for decomposing a polyol or a polyol ester.

### BACKGROUND ART

In order to suppress consumption of natural resources and reduce burden on the environment, organic substances contained in waste are reused as energy resources and chemical raw materials. For example, Patent Literature 1 describes a method for producing terephthalic acid and ethylene glycol by hydrolyzing polyesters, polyamides, and polycarbonates contained in waste in an environment of 200 °C to 300 °C and a pressure of 15 atmospheres or more using water as a medium.

Further, biomass (renewable biologically-derived organic resources excluding fossil resources) including leftover food and beer lees, sawdust and livestock manure, old clothes and used paper is utilized as fuel, feed, and compost, and may also be utilized in the production of saccharides. For example, Patent Literature 2 describes a method for producing a polysaccharide by repeating a step of subjecting an extraction residue of coffee beans to a heat treatment in the presence of a dilute alkali and a step of allowing cellulase to act on the extraction residue.

### CITATION LIST

### PATENT LITERATURE

Patent Literature 1: US 4605762 B
Patent Literature 2: JP 2007-217466 A
Patent Literature 3: JP 2015-157792 A

### NON PATENT LITERATURE

Non Patent Literature 1: Duncan, S.M.; Alkasrawi, M.; Gurram, R.; Almomani, F.; Wiberley-Bradford, A.E.; Singsaas, E. Paper Mill Sludge as a Source of Sugars for Use in the Production of Bioethanol and Isoprene. Energies 2020, 13, 4662.

Non Patent Literature 2: Meadows S, Hosur M, Celikbag Y, Jeelani S. Comparative Analysis on the Epoxidation of Soybean Oil using Formic and Acetic Acids. Polymers and Polymer Composites. 2018;26(4):289-298.

Non Patent Literature 3: Ismail, Dandi N. and Nazlee Faisal Ghazali. "SEPARATION OF FATTY ACIDS FROM PALM OIL USING ORGANIC SOLVENT NANOFILTRATION." Malaysian Journal of Analytical Science (2018): n. pag.

Non Patent Literature 4: M S Sinaga et al 2017 IOP Conf. Ser.: Mater. Sci. Eng. 180 012125

Non Patent Literature 5: Mohammad, S.; Baidurah, S.; Kobayashi, T.; Ismail, N.; Leh, C.P. Palm Oil Mill Effluent Treatment Processes - A Review. Processes 2021, 9, 739.

### SUMMARY OF INVENTION

### TECHNICAL PROBLEM

In the method described in Patent Literature 1, a powerful heat source is required in order to raise the temperature of water to a high temperature of 200 °C to 300 °C, which may actually increase the burden on the environment. In the method of Patent Literature 2, it is necessary to repeat dilute alkali treatment and enzyme treatment, making the process troublesome.

The organic substances contained in waste are varied, but are often classified as polyols or polyol esters. Since the polyols and the polyol esters remaining as waste have a chemical structure that makes them difficult to biodegrade, these decomposition reactions by enzymes and microorganisms are unstable and slow.

The problem to be solved by the present invention is to provide a method for more easily decomposing a polyol or a polyol ester.

### SOLUTION TO PROBLEM

A method for decomposing a polyol or a polyol ester according to the present invention, which has been made to solve the above problems, includes
bringing, in predetermined reaction water, a substance to be decomposed containing at least one of a polyol and a polyol ester into contact with a solid of a metal oxide or a metal hydroxide having a standard oxidation-reduction potential different from that of water.

In the present description, the "polyol" is a compound having two or more hydroxy groups in its molecule, and the "polyol ester" is a compound obtained by esterifying a polyol.

Examples of the metal oxide and the metal hydroxide having a standard oxidation-reduction potential different from that of water can include alumina (Al₂O₃), silica (SiO₂), zeolite (Al₂O₃·SiO₂), titania (TiO₂), vanadium pentoxide (V₂O₅), manganese dioxide (MnO₂), iron hydroxide (FeO(OH)), zinc oxide (ZnO), germanium oxide (GeO₂), tin oxide (SnO₂), lead oxide (PbO₂), nickel oxide (NiO₂, Ni₃O₄), nickel hydroxide (Ni(OH)₃), and mixtures of two or more these. Of course, other than these, metal oxides and metal hydroxides having standard oxidation-reduction potentials different from that of water can also be used.

In the method for decomposing a polyol or a polyol ester according to the present invention, in order to increase a rate of a hydrolysis reaction to be described later, it is preferable to heat the substance to be decomposed to 100 °C or higher in a state of being in contact with a solid of a metal oxide or a metal hydroxide in predetermined reaction water.

The solid of the metal oxide and the metal hydroxide may have any shape as long as its surface can be brought into contact with the substance to be decomposed (polyol or polyol ester), but is preferably in a form of particles, powder, or the like in order to increase a contact area with the substance to be decomposed to increase the rate of the hydrolysis reaction to be described later. Further, the substance to be decomposed may also have any shape, but is preferably in the form of small pieces (chips) in order to increase the contact area with the solid of the metal oxide and the metal hydroxide to increase the rate of the hydrolysis reaction to be described later.

The reaction water is typically pure water (1 MΩ·cm or more) or ultrapure water (18.21 MΩ·cm or more), but can also be industrial purified water (typically 10 kΩ·cm or more) or industrial wastewater (typically 10 kΩ·cm or less). Further, the reaction water is typically neutral (pH = 6 to 8), but may contain an acid (sulfuric acid, phosphoric acid, carbonic acid, and the like), an alkali or a salt (sodium hydroxide, potassium hydroxide, calcium hydroxide, calcium oxide, sodium carbonate, sodium hydrogen carbonate, and the like), or a water-soluble oxide (calcium hypochlorite, sodium percarbonate, and the like) that have been conventionally used for hydrolyzing a polymer compound.

### ADVANTAGEOUS EFFECTS OF INVENTION

In the present invention, the polyol or the polyol ester can be decomposed by bringing, in predetermined reaction water, a substance to be decomposed containing at least one of the polyol and the polyol ester into contact with a solid of a metal oxide or a metal hydroxide having a standard oxidation-reduction potential different from that of water.

The mechanism by which such a reaction occurs is not completely clear at the present time, and it is considered that the mechanism varies depending on the type of metal oxide and metal hydroxide, but it is presumed that the mechanism is due to a difference in standard oxidation-reduction potential. In general, a reductant of a system having a lower standard oxidation-reduction potential is more likely to release electrons, and an oxidant of a system having a higher standard oxidation-reduction potential is more likely to accept electrons. Therefore, it is presumed that on the surface of the solid of the metal oxide or the metal hydroxide having a standard oxidation-reduction potential higher than that of water, water molecules (H₂O) are spontaneously oxidized to generate protons (H⁺), electrons (e⁻), and oxygen (O₂), and the solid surface (solid-liquid interface) of the metal oxide or the metal hydroxide becomes a strong acidic environment. Further, it is presumed that on the surface of the solid of the metal oxide or the metal hydroxide having a standard oxidation-reduction potential lower than that of water, water molecules (H₂O) are spontaneously reduced to generate hydrogen (H₂) and hydroxide ions (OH⁻), and the solid surface (solid-liquid interface) of the metal oxide or the metal hydroxide becomes a strong alkaline environment.

However, it is considered that a strong acidic environment or alkaline environment occurs only near the solid-liquid interface, and the entire water (bulk) does not become an acidic environment or an alkaline environment. Therefore, in the present invention, a substance to be decomposed containing at least one of the polyol and the polyol ester is brought into contact with the surface of a solid of a metal oxide or a metal hydroxide in predetermined reaction water. As a result, the substance to be decomposed is effectively hydrolyzed.

As described above, according to the method for decomposing a polyol or a polyol ester according to the present invention, by bringing, in predetermined reaction water, a substance to be decomposed containing at least one of a polyol and a polyol ester into contact with a solid of a metal oxide or a metal hydroxide having a standard oxidation-reduction potential different from that of water, the polyol or the polyol ester can be more easily decomposed.

### BRIEF DESCRIPTION OF DRAWINGS

Fig. 1 is a diagram showing a polyol and a polyol ester.
Fig. 2 is a diagram schematically showing a hydrolysis reaction according to an embodiment.
Fig. 3 is a chromatogram of components contained in a filtrate obtained in Experiment 48.
Fig. 4 is a chromatogram of components contained in a filtrate obtained in Experiment 49.
Fig. 5 is a chromatogram of components contained in a filtrate obtained in Experiment 50.
Fig. 6 is an XRD spectrum of a substance attached to the surface of lead oxide remaining on a filter.
Fig. 7 is an XRD spectrum of commercially available disodium terephthalate.
Fig. 8 is an XRD spectrum of lead oxide powder remaining on the filter.
Fig. 9 is an XRD spectrum of unused lead oxide powder.
Fig. 10 is an SEM observation image of unused lead oxide.
Fig. 11 is a chromatogram of components contained in a filtrate obtained in Experiment 51 when a reaction time was set to 1 hour.
Fig. 12 is a chromatogram of components contained in a filtrate obtained in Experiment 51 when the reaction time was set to 3 hours.
Fig. 13 is a chromatogram of components contained in a filtrate obtained in Experiment 51 when the reaction time was set to 6 hours.
Fig. 14 is a chromatogram of components contained in a filtrate obtained in a comparative experiment.
Fig. 15 is an XRD spectrum of a substance attached to the surface of lead oxide remaining on the filter.
Fig. 16 is an XRD spectrum of commercially available disodium terephthalate.

### DESCRIPTION OF EMBODIMENTS

An exemplary embodiment of the method for decomposing a polyol or a polyol ester according to the present invention will be described. In the present embodiment, a substance to be decomposed containing at least one of a polyol and a polyol ester, and a solid of a metal oxide or a metal hydroxide having a standard oxidation-reduction potential different from that of water, are placed in a reaction container containing water and brought into contact with each other.

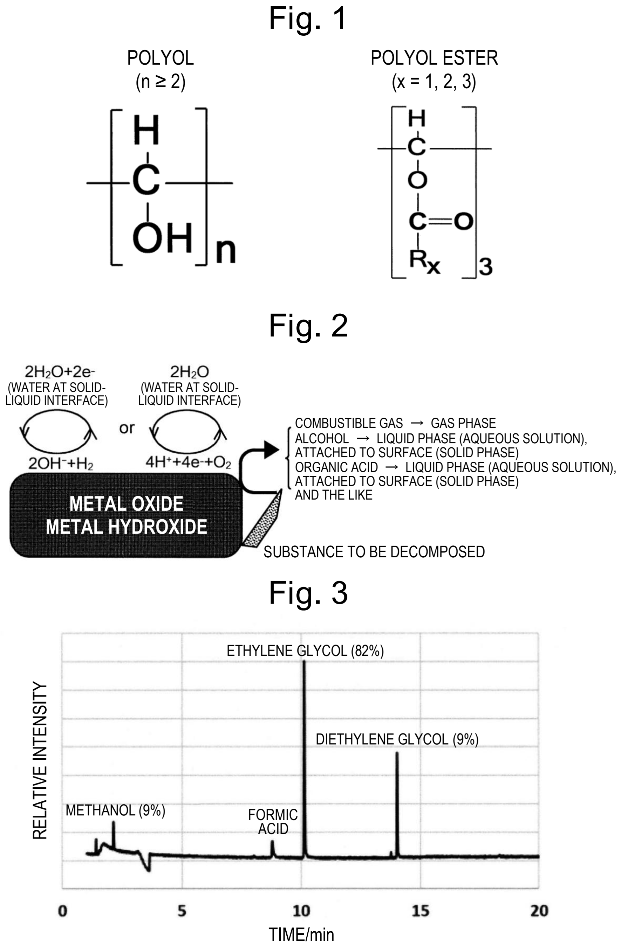

The substance to be decomposed in the present embodiment is any polyol or polyol ester as shown in Fig. 1, and is, for example, glucose, starch, cellulose, galactose, mannose, arabinose, fructose, sucrose, maltose, lactose, cellobiose, pectin, lignin, xylan, xylose, glycerol, triacetin, glyceride (glyceride, acyl glycerol, vegetable oil, edible oil, lubricating oil for machines, etc.), polyvinyl alcohol, polyester, ethylene glycol, or a mixture of two or more these. The reaction water used in the present embodiment is typically pure water (1 MΩ·cm or more) or ultrapure water (18.21 MΩ·cm or more), but can also be industrial purified water (typically 10 kΩ·cm or more) or industrial wastewater (typically 10 kΩ·cm or less). Further, the reaction water used in the present embodiment is typically neutral (pH = 6 to 8), but may contain an acid (sulfuric acid, phosphoric acid, carbonic acid, and the like), an alkali or a salt (sodium hydroxide, potassium hydroxide, calcium hydroxide, calcium oxide, sodium carbonate, sodium hydrogen carbonate, and the like), or a water-soluble oxide (calcium hypochlorite, sodium percarbonate, and the like) that has been conventionally used for hydrolyzing a polymer compound. Examples of the metal oxide and the metal hydroxide used in the present embodiment that have a standard oxidation-reduction potential different from that of water can include alumina (Al₂O₃), silica (SiO₂), zeolite (Al₂O₃·SiO₂), titania (TiO₂), vanadium pentoxide (V₂O₅), manganese dioxide (MnO₂), iron hydroxide (FeO(OH)), zinc oxide (ZnO), germanium oxide (GeO₂), tin oxide (SnO₂), lead oxide (PbO₂), nickel oxide (NiO₂, Ni₃O₄), nickel hydroxide (Ni(OH)₃), and mixtures of two or more these. Of course, other than these, metal oxides and metal hydroxides having standard oxidation-reduction potentials different from that of water can also be used. The decomposition method according to the present embodiment can be performed by arbitrarily combining the substances to be decomposed, the reaction water, the metal oxide, and the metal hydroxide exemplified above.

In the method for decomposing a polyol or a polyol ester according to the present embodiment, in order to increase the rate of hydrolysis reaction, it is preferable to heat the substance to be decomposed to 100 °C or higher in a state of being in contact with the solid of the metal oxide or the metal hydroxide in predetermined reaction water. In particular, when a monosaccharide, being a representative polyol, is heated to 100 °C or higher, the monosaccharide may decompose exothermically singly, causing the rate of the decomposition reaction to dramatically increase.

The solid of the metal oxide and the metal hydroxide may have any shape as long as its surface can be brought into contact with the substance to be decomposed (polyol or polyol ester), but is preferably in the form of particles, powder, or the like in order to increase the contact area with the substance to be decomposed to increase the rate of the hydrolysis reaction. Further, the substance to be decomposed may also have any shape, but is preferably in the form of small pieces (chips) in order to increase the contact area with the solid of the metal oxide and the metal hydroxide to increase the rate of the hydrolysis reaction. In the decomposition method according to the present embodiment, either one of the substance to be decomposed and the solid of the metal oxide and the metal hydroxide may have the above shape, or both of them may have the above shape.

The reaction container used in the present embodiment can be any known type, and is preferably a sealed container. The inside of the reaction container may be in an air atmosphere or a vacuum atmosphere. Further, in the present embodiment, as a heating method, for example, an electric heater, a heating medium such as water vapor or oil, or a microwave can be used.

In the present embodiment, the reaction and the standard oxidation-reduction potential (E°) of water (H₂O) in the reaction container are as follows (1) and (2). Further, as an example of the reaction and the standard oxidation-reduction potential of the solid of the metal oxide or the metal hydroxide used in the present embodiment, the respective reactions and the standard oxidation-reduction potentials of lead oxide, nickel oxide, and nickel hydroxide are shown below in (3) to (6).
(1) 2H₂O (liquid) ⇔ 4H⁺ + 4e⁻ + O₂ E° = 1.229 V
(2) 2H₂O (liquid) + 2e⁻ ⇔ H₂ + 2OH⁻ E° = -0.83 V
(3) PbO₂ (solid) + 4H⁺ + 2e⁻ ⇔ Pb²⁺ + 2H₂O E° = 1.468 V
(4) NiO₂ (solid) + 4H⁺ + 2e⁻ ⇔ Ni²⁺ + 2H₂O E° = 1.68 V
(5) Ni₃O₄ (solid) + 8H⁺ + 2e⁻ ⇔ 3Ni²⁺ + 4H₂O E° = 1.977 V
(6) Ni(OH)₃ (solid) + 3H⁺ + e⁻ ⇔ Ni²⁺ + 3H₂O E° = 2.08 V

In the reaction equations shown in the above (1) to (6), bidirectional arrow symbols (⇔) represent equilibrium.

In a case where two or more chemical equilibria consisting of any one or more of the above (1) and (3) to (6) exist simultaneously, a reductant of a system having a lower standard oxidation-reduction potential is more likely to release electrons, and an oxidant of a system having a higher standard oxidation-reduction potential is more likely to accept electrons. Therefore, it is considered that on the surface of the solid of the metal oxide and the metal hydroxide having a standard oxidation-reduction potential higher than that of water, the reaction of the above (1) spontaneously proceeds to the right, and the solid surface (solid-liquid interface) of the metal oxide or the metal hydroxide becomes a strong acidic environment. In this state, as shown in Fig. 2, the substance to be decomposed containing at least one of the polyol and the polyol ester in contact with the surface of the solid of the metal oxide or the metal hydroxide is hydrolyzed, and industrially useful chemical components such as a combustible gas, an alcohol, and an organic acid are easily obtained.

The same applies to metal oxides and metal hydroxides having a higher standard oxidation-reduction potential than that of water, other than the metal oxides and the metal hydroxides shown in the above (3) to (6). Further, it is considered that on the surface of the solid of the metal oxide and the metal hydroxide having a standard oxidation-reduction potential lower than that of water, the reaction of the above (2) spontaneously proceeds to the right, and the solid surface (solid-liquid interface) of the metal oxide or the metal hydroxide becomes a strong alkaline environment.

A measured value of the standard oxidation-reduction potential may vary depending on a measurement method. In the present embodiment, the value of the standard oxidation-reduction potential may be measured by any known measurement method. Based on the values of (1) and (2) above, embodiments of the present invention include a case where the standard oxidation-reduction potential of the metal oxide or the metal hydroxide is less than -0.83 V and a case where the standard oxidation-reduction potential is more than 1.229 V.

Note that an upper limit of a heating temperature in the present embodiment is 375 °C, which is the critical point of water, but the heating can be typically performed at 100 °C to 200 °C. In the method described in Patent Literature 1, a powerful heat source is required in order to raise the temperature of water to a high temperature of 200 °C to 300 °C, whereas in the present embodiment, the method can be performed at a lower temperature, so that the burden on the environment associated with securing a heat source can be reduced.

### EXAMPLE

Hereinafter, as examples of the method for decomposing a polyol or a polyol ester according to the present embodiment, a plurality of results of experiments for confirming effects of the method for decomposing a polyol or a polyol ester according to the present embodiment will be described. The types and amounts of substances produced in each experiment were measured using various instruments. In other words, a concentration of hydrogen (H₂) gas was measured with XP-3340 (2) (numbers in parentheses are Roman numerals) manufactured by NEW COSMOS ELECTRIC CO., LTD. or a Kitagawa type gas detection tube manufactured by GASTEC CORPORATION, the concentration of carbon monoxide (CO) gas was measured with a Kitagawa type gas detection tube manufactured by GASTEC CORPORATION, and the concentration of methane (CH₄) gas was measured with GX-6000 manufactured by RIKEN KEIKI Co., Ltd. The concentrations of water-soluble components such as lower alcohols, acetones, aldehydes, and organic acids were quantified by measuring components contained in a filtrate obtained by removing metal oxides or metal hydroxides through suction filtration using a filter (pore size 0.025 µm, diameter 47 mm) manufactured by Merck Millipore simultaneously with a standard substance using a gas chromatograph mass spectrometer (GC-MS) GCMS-QP2010Ultra manufactured by Shimadzu Corporation. The water-insoluble components such as higher alcohols and light oils attached to metal oxides or metal hydroxides, were quantified by drying the metal oxides or metal hydroxides after the suction filtration at 80 °C and then extracting with hexane, and measuring the hexane solution in which the water-insoluble component was dissolved simultaneously with the standard substance using the GC-MS.

Note that, in all the measurements, measurement conditions of a gas chromatograph (GC) part and a mass spectrometry (MS) part in the GC-MS were set as follows.
(1) GC Part
   Carrier Gas: Helium (He) (linear velocity 40 cm/sec, split ratio 50)
   Column: DB-WAX UI (30 m, diameter 0.25 mm, film thickness 0.25 µm) manufactured by Agilent Technologies Japan, Ltd.
   Injection temperature: 220 °C
   Column temperature: 50 °C to 200 °C (10 °C/min)
(2) MS Part
   Interface temperature: 220 °C
   Ion source temperature: 200 °C
   Measurement mode: Scan mode (mass-to-charge ratio 25-300)

### [Experiment 1]

In Experiment 1, 1.0348 g (0.0057 mol) in weight of glucose (approximately 180 g/mol, manufactured by NACALAI TESQUE, INC.), 9.07 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of glucose is 0.115 mol/L. In this state, a decomposition reaction was carried out at 200 °C for 1 hour while stirring at a rotation speed of 200 rpm. As a result, from the gas phase, 0.00233 mol (40.5 mol%) of hydrogen and 0.00004 mol (0.7 mol%) of carbon monoxide were obtained, and from the liquid phase, as aqueous solutions, 0.15 mol/L of acetic acid (130 mol%, where mol% is the ratio of the amount of substance of a product (acetic acid) to the amount of substance of a raw material (glucose). The same applies hereinafter in the present specification.), 0.012 mol/L (10.1 mol%) of formic acid, 0.0041 mol/L (3.6 mol%) of ethylene glycol, 0.0025 mol/L (2.16 mol%) of diethylene glycol, and trace amounts of acetone, methanol, and ethanol were obtained, respectively.

### [Experiment 2]

In Experiment 2, 2.08 g (0.012 mol) in weight of glucose (approximately 180 g/mol, manufactured by NACALAI TESQUE, INC.), 4 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (MMJ-100 manufactured by OMLABO TECH CO., LTD., capacity 120 mL), and the container was sealed. At this point, the concentration of glucose is 0.23 mol/L. In this state, a decomposition reaction was carried out at 250 °C for 1 hour while stirring at a rotation speed of 400 rpm. As a result, from the gas phase, 0.0058 mol (50.4 mol%) of hydrogen and 0.0005 mol (0.4 mol%) of carbon monoxide were obtained, and from the liquid phase, as aqueous solutions, 0.0083 mol/L (3.6 mol%) of acetone, 0.0019 mol/L (0.87 mol%) of methanol, 0.0026 mol/L (1.1 mol%) of ethanol, 0.016 mol/L (6.7 mol%) of acetic acid, 0.0020 mol/L (0.88 mol%) of formic acid, 0.0018 mol/L (0.77 mol%) of ethylene glycol, and 0.00033 mol/L (0.14 mol%) of diethylene glycol were obtained.

### [Experiment 3]

In Experiment 3, 1.0364 g (0.0058 mol) in weight of glucose (approximately 180 g/mol, manufactured by NACALAI TESQUE, INC.), 9.13 g of tin oxide (SnO₂, manufactured by FUJIFILM Wako Pure Chemical Corporation), and 50 mL of industrial purified water were placed in a pressure-resistant container (TEM-D100M manufactured by Taiatsu Techno Corporation, capacity 128 mL), and the container was sealed. At this point, the concentration of glucose is 0.115 mol/L. In this state, a decomposition reaction was carried out at 200 °C for 1 hour while stirring at a rotation speed of 200 rpm. As a result, from the gas phase, 0.000404 mol (7.02 mol%) of hydrogen and 0.00000505 mol (0.088 mol%) of carbon monoxide were obtained, and from the liquid phase, as aqueous solutions, 0.039 mol/L (33.7 mol%) of acetic acid, 0.00183 mol/L (1.59 mol%) of formic acid, 0.00147 mol/L (1.27 mol%) of ethylene glycol, 0.013 mol/L (1.1 mol%) of acetone, 0.00023 mol/L (0.2 mol%) of 1-propanol, 0.0087 mol/L (7.5 mol%) of 2-propanol, 0.001 mol/L (0.9 mol%) of acetoin, 0.009 mol/L (7.8 mol%) of hydroxyacetone, 0.0001 mol/L (0.09 mol%) of levulinic acid, 0.00029 mol/L (0.025 mol%) of furfural, and 0.00081 mol/L (0.07 mol%) of 5-hydroxymethylfurfural were obtained.

### [Experiment 4]

In Experiment 4, 1.0603 g (0.0059 mol) in weight of glucose (approximately 180 g/mol, manufactured by NACALAI TESQUE, INC.), 9.07 g of germanium oxide (GeO₂, manufactured by NACALAI TESQUE, INC.), and 50 mL of industrial purified water were placed in a pressure-resistant container (TEM-D100M manufactured by Taiatsu Techno Corporation, capacity 128 mL), and the container was sealed. At this point, the concentration of glucose is 0.118 mol/L. In this state, a decomposition reaction was carried out at 200 °C for 1 hour while stirring at a rotation speed of 200 rpm. As a result, from the gas phase, 0.0000504 mol (0.856 mol%) of hydrogen and 0.00000403 mol (0.068 mol%) of carbon monoxide were obtained, and from the liquid phase, as aqueous solutions, 0.0014 mol/L (1.2 mol%) of methanol, 0.0025 mol/L (2.1 mol%) of ethanol, 0.012 mol/L (10.3 mol%) of acetic acid, 0.00713 mol/L (6.03 mol%) of formic acid, 0.0014 mol/L (1.2 mol%) of ethylene glycol, 0.0021 mol/L (1.8 mol%) of acetone, 0.00022 mol/L (0.18 mol%) of 1-propanol, 0.0035 mol/L (3 mol%) of 2-propanol, 0.00057 mol/L (0.49 mol%) of hydroxyacetone, 0.00082 mol/L (0.7 mol%) of furfural, and 0.0002 mol/L (0.17 mol%) of 5-hydroxymethylfurfural were obtained.

### [Experiment 5]

In Experiment 5, 1.0800 g (0.006 mol) in weight of glucose (180 g/mol, manufactured by NACALAI TESQUE, INC.), 9.04 g of silicon oxide (SiO₂, manufactured by FUJIFILM Wako Pure Chemical Corporation), and 50 mL of industrial purified water were placed in a pressure-resistant container (TEM-D100M manufactured by Taiatsu Techno Corporation, capacity 128 mL), and the container was sealed. At this point, the concentration of glucose is 0.12 mol/L. In this state, a decomposition reaction was carried out at 200 °C for 1 hour while stirring at a rotation speed of 200 rpm. As a result, from the gas phase, 0.000032 mol (0.54 mol%) of carbon monoxide was obtained, and from the liquid phase, as aqueous solutions, 0.0014 mol/L (1.2 mol%) of methanol, 0.0014 mol/L (1.2 mol%) of ethanol, 0.011 mol/L (9.5 mol%) of acetic acid, 0.011 mol/L (9.5 mol%) of formic acid, 0.00068 mol/L (0.56 mol%) of ethylene glycol, 0.0016 mol/L (1.3 mol%) of acetone, 0.00027 mol/L (0.23 mol%) of acetoin, 0.0015 mol/L (1.2 mol%) of hydroxyacetone, 0.0017 mol/L (1.4 mol%) of furfural, and 0.0005 mol/L (0.4 mol%) of 5-hydroxymethylfurfural were obtained.

### [Experiment 6]

In Experiment 6, 1.0127 g (0.006 mol) in weight of glucose (approximately 180 g/mol, manufactured by NACALAI TESQUE, INC.), 9.03 g of alumina (Al₂O₃, manufactured by Mizusawa Industrial Chemicals, Ltd.), and 50 mL of industrial purified water were placed in a pressure-resistant container (TEM-D100M manufactured by Taiatsu Techno Corporation, capacity 128 mL), and the container was sealed. At this point, the concentration of glucose is 0.11 mol/L. In this state, a decomposition reaction was carried out at 200 °C for 1 hour while stirring at a rotation speed of 200 rpm. As a result, from the gas phase, 0.000015 mol (0.26 mol%) of carbon monoxide was obtained, and from the liquid phase, as aqueous solutions, 0.0076 mol/L (6.6 mol%) of acetic acid, 0.0026 mol/L (2.4 mol%) of formic acid, 0.0000895 mol/L (0.0785 mol%) of ethylene glycol, 0.00096 mol/L (0.85 mol%) of acetoin, 0.0097 mol/L (8.5 mol%) of hydroxyacetone, 0.00025 mol/L (0.2 mol%) of furfural, 0.0005 mol/L (0.4 mol%) of 5-hydroxymethylfurfural were obtained, and as components of a chromatographic peak of GCMS whose concentration was not determined but was clear, dihydroxyacetone, 2-hydroxy-γ-butyrolactone, and levulinic acid were obtained, respectively.

### [Experiment 7]

In Experiment 7, 1.0442 g (0.0058 mol) in weight of glucose (approximately 180 g/mol, manufactured by NACALAI TESQUE, INC.), 9.04 g of anatase type titanium oxide (TiO₂, manufactured by Tokyo Chemical Industry Co., Ltd.), and 50 mL of industrial purified water were placed in a pressure-resistant container (TEM-D100M manufactured by Taiatsu Techno Corporation, capacity 128 mL), and the container was sealed. At this point, the concentration of glucose is 0.12 mol/L. In this state, a decomposition reaction was carried out at 200 °C for 1 hour while stirring at a rotation speed of 200 rpm. As a result, from the gas phase, 0.00040 mol (7 mol%) of hydrogen and 0.000013 mol (0.22 mol%) of carbon monoxide were obtained, and from the liquid phase, as aqueous solutions, 0.0011 mol/L (0.97 mol%) of methanol, 0.00094 mol/L (0.81 mol%) of acetic acid, 0.011 mol/L (9.4 mol%) of formic acid, 0.000036 mol/L (0.031 mol%) of acetoin, 0.00032 mol/L (0.28 mol%) of hydroxyacetone, 0.00043 mol/L (0.37 mol%) of furfural, and 0.00023 mol/L (0.2 mol%) of 5-hydroxymethylfurfural were obtained.

### [Experiment 8]

In Experiment 8, 1.0065 g (0.0056 mol) in weight of glucose (approximately 180 g/mol, manufactured by NACALAI TESQUE, INC.), 9.02 g of rutile type titanium oxide (TiO₂, manufactured by FUJIFILM Wako Pure Chemical Corporation), and 50 mL of industrial purified water were placed in a pressure-resistant container (TEM-D100M manufactured by Taiatsu Techno Corporation, capacity 128 mL), and the container was sealed. At this point, the concentration of glucose is 0.11 mol/L. In this state, a decomposition reaction was carried out at 200 °C for 1 hour while stirring at a rotation speed of 200 rpm. As a result, from the gas phase, 0.00005 mol (0.9 mol%) of carbon monoxide was obtained, and from the liquid phase, as aqueous solutions, 0.0011 mol/L (0.98 mol%) of methanol, 0.0042 mol/L (3.8 mol%) of acetic acid,0.00043 mol/L (0.39 mol%) of formic acid, 0.000031 mol/L (0.028 mol%) of ethylene glycol, 0.00017 mol/L (0.15 mol%) of acetoin, 0.0020 mol/L (1.8 mol%) of hydroxyacetone, 0.001 mol/L (0.9 mol%) of levulinic acid, 0.001 mol/L (0.9 mol%) of furfural, and 0.000058 mol/L (0.052 mol%) of 5-hydroxymethylfurfural were obtained.

### [Experiment 9]

In Experiment 9, 1 g (0.0056 mol) in weight of glucose (approximately 180 g/mol, manufactured by NACALAI TESQUE, INC.), 8 g of vanadium pentoxide (V₂O₅, manufactured by KANTO CHEMICAL CO., INC.), and 50 mL of ultrapure water (specific resistance 18.2 MΩ·cm, manufactured by ELGA) were placed in a pressure-resistant container (NR0218 manufactured by Flonchemical Co., Ltd., capacity 100 mL), and the container was sealed. At this point, the concentration of glucose is 0.11 mol/L. In this state, the decomposition reaction was carried out at 200 °C for 2 hours while stirring at a rotation speed of 200 rpm. As a result, from the gas phase, 0.00082 mol (14.8 mol%) of hydrogen and 0.0003 mol (5 mol%) of carbon monoxide were obtained, and from the liquid phase, as aqueous solutions, 0.03 mol/L (26.6 mol%) of acetic acid, 0.011 mol/L (10 mol%) of formic acid, 0.00037 mol/L (0.34 mol%) of ethylene glycol, and 0.00076 mol/L (0.69 mol%) of diethylene glycol were obtained.

### [Experiment 10]

In Experiment 10, 1 g (0.0056 mol) in weight of glucose (approximately 180 g/mol, manufactured by NACALAI TESQUE, INC.), 8 g of vanadium pentoxide (V₂O₅, manufactured by KANTO CHEMICAL CO., INC.), 50 mL of ultrapure water (specific resistance 18.2 MΩ·cm, manufactured by ELGA), and 100 µL of 1 mol/L sulfuric acid were placed in a pressure-resistant container (NR0218 manufactured by Flonchemical Co., Ltd., capacity 100 mL), and the container was sealed. At this point, the concentration of glucose is 0.11 mol/L and the pH is about 2. In this state, the decomposition reaction was carried out at 200 °C for 2 hours while stirring at a rotation speed of 200 rpm. As a result, from the gas phase, 0.00082 mol (14.8 mol%) of hydrogen and 0.00041 mol (7.4 mol%) of carbon monoxide were obtained, and from the liquid phase, as aqueous solutions, 0.0024 mol/L (2.1 mol%) of methanol, 0.0027 mol/L (2.4 mol%) of acetic acid, 0.0031 mol/L (2.8 mol%) of formic acid, and 0.0014 mol/L (1.3 mol%) of ethylene glycol were obtained.

### [Experiment 11]

In Experiment 11, 1 g (0.0056 mol) in weight of glucose (approximately 180 g/mol, manufactured by NACALAI TESQUE, INC.), 8 g of vanadium pentoxide (V₂O₅, manufactured by KANTO CHEMICAL CO., INC.), 50 mL of ultrapure water (specific resistance 18.2 MΩ·cm, manufactured by ELGA), and 100 µL of 1 mol/L sodium hydroxide were placed in a pressure-resistant container (NR0218 manufactured by Flonchemical Co., Ltd., capacity 100 mL), and the container was sealed. At this point, the concentration of glucose is 0.11 mol/L and the pH is about 12. In this state, the decomposition reaction was carried out at 200 °C for 2 hours while stirring at a rotation speed of 200 rpm. As a result, from the gas phase, 0.00029 mol (5.2 mol%) of hydrogen and 0.00017 mol (3.1 mol%) of carbon monoxide were obtained, and from the liquid phase, as aqueous solutions, 0.0016 mol/L (1.4 mol%) of methanol, 0.053 mol/L (48 mol%) of acetic acid and 0.13 mol/L (119 mol%) of formic acid were obtained.

### [Experiment 12]

1 g (0.0056 mol) in weight of glucose (approximately 180 g/mol, manufactured by NACALAI TESQUE, INC.), 8 g of manganese dioxide (MnO₂, manufactured by KANTO CHEMICAL CO., INC.), and 50 mL of ultrapure water (specific resistance 18.2 MΩ·cm, manufactured by ELGA) were placed in a pressure-resistant container (NR0218 manufactured by Flonchemical Co., Ltd., capacity 100 mL), and the container was sealed. At this point, the concentration of glucose is 0.11 mol/L. In this state, the decomposition reaction was carried out at 200 °C for 2 hours while stirring at a rotation speed of 200 rpm. As a result, from the gas phase, 0.00040 mol (7.2 mol%) of hydrogen and 0.00013 mol (2.4 mol%) of carbon monoxide were obtained, and from the liquid phase, as aqueous solutions, 0.0012 mol/L (1.1 mol%) of acetone, 0.0013 mol/L (1.2 mol%) of methanol, 0.020 mol/L (18.4 mol%) of ethanol, and 0.022 mol/L (20.2 mol%) of acetic acid were obtained.

### [Experiment 13]

In Experiment 13, 1 g (0.0056 mol) in weight of glucose (approximately 180 g/mol, manufactured by NACALAI TESQUE, INC.), 8 g of manganese dioxide (MnO₂, manufactured by KANTO CHEMICAL CO., INC.), 50 mL of ultrapure water (specific resistance 18.2 MΩ·cm, manufactured by ELGA), and 100 µL of 1 mol/L sulfuric acid were placed in a pressure-resistant container (NR0218 manufactured by Flonchemical Co., Ltd., capacity 100 mL), and the container was sealed. At this point, the concentration of glucose is 0.11 mol/L and the pH is about 2. In this state, the decomposition reaction was carried out at 200 °C for 2 hours while stirring at a rotation speed of 200 rpm. As a result, from the gas phase, 0.00082 mol (14.8 mol%) of hydrogen and 0.00033 mol (6 mol%) of carbon monoxide were obtained, and from the liquid phase, as aqueous solutions, 0.0058 mol/L (5.3 mol%) of methanol, 0.011 mol/L (10.1 mol%) of acetic acid and 0.0035 mol/L (3.1 mol%) of formic acid were obtained.

### [Experiment 14]

In Experiment 14, 1 g (0.0056 mol) in weight of glucose (approximately 180 g/mol, manufactured by NACALAI TESQUE, INC.), 8 g of manganese dioxide (MnO₂, manufactured by KANTO CHEMICAL CO., INC.), 50 mL of ultrapure water (specific resistance 18.2 MΩ·cm, manufactured by ELGA), and 100 µL of 1 mol/L sodium hydroxide were placed in a pressure-resistant container (NR0218 manufactured by Flonchemical Co., Ltd., capacity 100 mL), and the container was sealed. At this point, the concentration of glucose is 0.11 mol/L and the pH is about 12. In this state, the decomposition reaction was carried out at 200 °C for 2 hours while stirring at a rotation speed of 200 rpm. As a result, from the gas phase, 0.00029 mol (5.2 mol%) of hydrogen and 0.00017 mol (3.1 mol%) of carbon monoxide were obtained, and from the liquid phase, as aqueous solutions, 0.0044 mol/L (3.9 mol%) of methanol, 0.011 mol/L (10.1 mol%) of acetic acid and 0.0065 mol/L (5.9 mol%) of formic acid were obtained.

### [Experiment 15]

In Experiment 15, 1.0318 g (0.0057 mol) in weight of glucose (approximately 180 g/mol, manufactured by NACALAI TESQUE, INC.), 9.0212 g of α-iron hydroxide (FeO(OH), manufactured by Kojundo Chemical Laboratory Co., Ltd.), and 50 mL of ultrapure water (specific resistance 18.2 MΩ·cm, manufactured by ELGA) were placed in a pressure-resistant container (TEM-D100M manufactured by Taiatsu Techno Corporation, capacity 100 mL), and the container was sealed. At this point, the concentration of glucose is 0.11 mol/L. In this state, the decomposition reaction was carried out at 200 °C for 4 hours while stirring at a rotation speed of 200 rpm. As a result, from the gas phase, 0.000025 mol (0.44 mol%) of hydrogen, 0.0000051 mol (0.089 mol%) of carbon monoxide, and 0.0000051 mol (0.089 mol%) of methane were obtained, and from the liquid phase, as aqueous solutions, 0.0033 mol/L (2.9 mol%) of acetone, 0.0035 mol/L (3.0 mol%) of methanol, 0.0026 mol/L (2.3 mol%) of ethanol, 0.0078 mol/L (6.8 mol%) of acetic acid, 0.0069 mol/L (6.0 mol%) of formic acid, and 0.00052 mol/L (0.46 mol%) of ethylene glycol were obtained, respectively. At the same time, hydroxyacetone, 2-methyl-2-cyclopentene, and propionic acid were also significantly detected as chromatographic peaks of GCMS.

### [Experiment 16]

In Experiment 16, 1.0040 g (0.0056 mol) in weight of glucose (approximately 180 g/mol, manufactured by NACALAI TESQUE, INC.), 9.0212 g of zinc oxide (ZnO, manufactured by Kojundo Chemical Lab. Co., Ltd.), and 50 mL of ultrapure water (specific resistance 18.2 MΩ·cm, manufactured by ELGA) were placed in a pressure-resistant container (TEM-D100M manufactured by Taiatsu Techno Corporation, capacity 100 mL), and the container was sealed. At this point, the concentration of glucose is 0.11 mol/L. In this state, the decomposition reaction was carried out at 200 °C for 2 hours while stirring at a rotation speed of 200 rpm. As a result, from the gas phase, 0.00075 mol (13.5 mol%) of hydrogen and 0.000015 mol (0.27 mol%) of carbon monoxide were obtained, and from the liquid phase, as aqueous solutions, 0.0026 mol/L (2.4 mol%) of acetone, 0.0018 mol/L (1.6 mol%) of methanol, 0.011 mol/L (10.0 mol%) of acetic acid, 0.0059 mol/L (5.3 mol%) of formic acid, and 0.002 mol/L (1.8 mol%) of ethylene glycol were obtained. At the same time, hydroxyacetone and acetoin were also significantly detected as chromatographic peaks of GCMS.

### [Experiment 17]

In Experiment 17, 1.07 g (0.0059 mol) in weight of glucose (approximately 180 g/mol, manufactured by NACALAI TESQUE, INC.), 5 g of zeolite (TOSOH HZSM-5 manufactured by Tosoh Corporation, Si/Al₂ = 21.6), and 50 mL of ultrapure water (specific resistance 18.2 MΩ·cm, manufactured by ELGA) were placed in a pressure-resistant container (MMJ-100 manufactured by OMLABO TECH CO., LTD., capacity 120 mL), and the container was sealed. At this point, the concentration of glucose is 0.12 mol/L. In this state, the decomposition reaction was carried out at 200 °C for 2 hours while stirring at a rotation speed of 200 rpm. As a result, from the gas phase, 0.0010 mol (16.9 mol%) of hydrogen and 0.00040 mol (6.8 mol%) of carbon monoxide were obtained, and from the liquid phase, as aqueous solutions, 0.0016 mol/L (1.3 mol%) of acetone, 0.0016 mol/L (1.4 mol%) of methanol, 0.0017 mol/L (1.4 mol%) of ethanol, 0.076 mol/L (6.4 mol%) of acetic acid, 0.043 mol/L (36.4 mol%) of formic acid, 0.0025 mol/L (2.1 mol%) of ethylene glycol, and 0.0016 mol/L (1.3 mol%) of diethylene glycol were obtained.

### [Experiment 18]

In Experiment 18, 2.01 g (0.011 mol) in weight of glucose (approximately 180 g/mol, manufactured by NACALAI TESQUE, INC.), 4.03 g of zeolite with a low silicon oxide ratio (TOSOH HZSM-5 manufactured by Tosoh Corporation, Si/Al₂ = 21.6), and 50 mL of ultrapure water (specific resistance 18.2 MΩ·cm, manufactured by ELGA) were placed in a pressure-resistant container (MMJ-100 manufactured by OMLABO TECH CO., LTD., capacity 120 mL), and the container was sealed. At this point, the concentration of glucose is 0.22 mol/L. In this state, a decomposition reaction was carried out at 250 °C for 1 hour while stirring at a rotation speed of 400 rpm. As a result, from the gas phase, 0.0010 mol (9 mol%) of hydrogen and 0.0030 mol (27 mol%) of carbon monoxide were obtained, and from the liquid phase, as aqueous solutions, 0.0031 mol/L (1.4 mol%) of acetone, 0.0019 mol/L (0.86 mol%) of methanol, 0.0019 mol/L (0.86 mol%) of ethanol, 0.013 mol/L (5.9 mol%) of acetic acid, 0.0061 mol/L (2.7 mol%) of formic acid, 0.0016 mol/L (0.72 mol%) of ethylene glycol, and 0.00032 mol/L (0.15 mol%) of diethylene glycol were obtained.

### [Experiment 19]

In Experiment 19, 2 g (0.011 mol) in weight of glucose (approximately 180 g/mol, manufactured by NACALAI TESQUE, INC.), 4.07 g of zeolite with a high silicon oxide ratio (HighSiZSM-5, Si/Al₂ = 148.2, provided by Nishiyama Lab., Division of Chemical Engineering, School of Engineering Science, Graduate School of Engineering Science, Osaka University), and 50 mL of ultrapure water (specific resistance 18.2 MΩ·cm, manufactured by ELGA) were placed in a pressure-resistant container (MMJ-100 manufactured by OMLABO TECH CO., LTD., capacity 120 mL), and the container was sealed. At this point, the concentration of glucose is 0.22 mol/L. In this state, a decomposition reaction was carried out at 250 °C for 1 hour while stirring at a rotation speed of 400 rpm. As a result, from the gas phase, 0.0017 mol (15.4 mol%) of hydrogen and 0.00040 mol (3.6 mol%) of carbon monoxide were obtained, and from the liquid phase, as aqueous solutions, 0.0030 mol/L (1.4 mol%) of acetone, 0.0018 mol/L (0.81 mol%) of methanol, 0.0020 mol/L (0.91 mol%) of ethanol, 0.013 mol/L (5.9 mol%) of acetic acid, 0.0032 mol/L (1.4 mol%) of formic acid, 0.0017 mol/L (0.75 mol%) of ethylene glycol, and 0.00032 mol/L (0.15mol%) of diethylene glycol were obtained.

### [Experiment 20]

In Experiment 20, 2.01 g (0.011 mol) in weight of glucose (approximately 180 g/mol, manufactured by NACALAI TESQUE, INC.), 1.04 g of lead oxide (PbO₂, manufactured by KANTO CHEMICAL CO., INC.), 4.06 g of zeolite (HighSiZSM-5, Si/Al₂ = 148.2, provided by Nishiyama Lab., Division of Chemical Engineering, School of Engineering Science, Graduate School of Engineering Science, Osaka University), and 50 mL of ultrapure water (specific resistance 18.2 MΩ·cm, manufactured by ELGA) were placed in a pressure-resistant container (MMJ-100 manufactured by OMLABO TECH CO., LTD., capacity 120 mL), and the container was sealed. At this point, the concentration of glucose is 0.22 mol/L. In this state, a decomposition reaction was carried out at 250 °C for 1 hour while stirring at a rotation speed of 400 rpm. As a result, from the gas phase, 0.0030 mol (27 mol%) of hydrogen and 0.000075 mol (0.67 mol%) of carbon monoxide were obtained, and from the liquid phase, as aqueous solutions, 0.0027 mol/L (1.2 mol%) of acetone, 0.0019 mol/L (0.85 mol%) of methanol, 0.0028 mol/L (1.3 mol%) of ethanol, 0.018 mol/L (8.1 mol%) of acetic acid, 0.0016 mol/L (0.71 mol%) of formic acid, 0.0018 mol/L (0.79 mol%) of ethylene glycol, and 0.00033 mol/L (0.15 mol%) of diethylene glycol were obtained.

Note that, Si/Al₂ characterizing the zeolite used in Experiments 17 to 20 is the ratio of the amount of substance of silicon and aluminum, and is calculated from data measured by the X-ray fluorescence analysis (XRF).

According to Experiments 1 to 20, glucose can be decomposed using various metal oxides, and industrially useful chemical components such as a combustible gas, an alcohol, an organic acid, a ketone, an aldehyde, a lactone, and an alkene can be easily obtained. Glucose is one of the most common organic compounds on the earth, and there are large amounts of waste and sludge containing glucose. By applying Experiments 1 to 20, there is a possibility that a social infrastructure can be constructed in which these wastes and sludge are efficiently converted into energy resources and chemical raw materials and recycled.

The substances to be decomposed, metal oxides or metal hydroxides, solvents, the presence or absence of acids, bases, and the like, and the types of products according to Experiments 1 to 20 are summarized in Tables 1 to 4.

**[Table 1-1]**

| No | Decomposition target | Metal oxide Metal hydroxide | Solvent | Acid/Base | Product | | |
|---|---|---|---|---|---|---|---|
| | | | | | Hydrogen | Carbon monoxide | Methane |
| Experiment 1 | Glucose | Lead oxide | Ultrapure water | | ○ | ○ | |
| Experiment 2 | Glucose | Lead oxide | Ultrapure water | | ○ | ○ | |
| Experiment 3 | Glucose | Tin oxide | Industrial purified water | | ○ | ○ | |
| Experiment 4 | Glucose | Germanium oxide | Industrial purified water | | ○ | ○ | |
| Experiment 5 | Glucose | Silicon oxide | Industrial purified water | | | ○ | |
| Experiment 6 | Glucose | Alumina | Industrial purified water | | | ○ | |
| Experiment 7 | Glucose | Anatase type titanium oxide | Industrial purified water | | ○ | ○ | |
| Experiment 8 | Glucose | Rutile type titanium oxide | Industrial purified water | | | ○ | |
| Experiment 9 | Glucose | Vanadium pentoxide | Ultrapure water | | ○ | ○ | |
| Experiment 10 | Glucose | Vanadium pentoxide | Ultrapure water | Sulfuric acid | ○ | ○ | |

**[Table 1-2]**

| No | Product | | | | | | |
|---|---|---|---|---|---|---|---|
| | Acetic acid | Formic acid | Ethylene glycol | Diethylene glycol | Acetone | Methanol | Ethanol |
| Experiment 1 | ○ | ○ | ○ | ○ | ○ | ○ | ○ |
| Experiment 2 | ○ | ○ | ○ | ○ | ○ | ○ | ○ |
| Experiment 3 | ○ | ○ | ○ | | ○ | | |
| Experiment 4 | ○ | ○ | ○ | | ○ | ○ | ○ |
| Experiment 5 | ○ | ○ | ○ | | ○ | ○ | ○ |
| Experiment 6 | ○ | ○ | ○ | | | | |
| Experiment 7 | ○ | ○ | | | | ○ | |
| Experiment 8 | ○ | ○ | ○ | | | ○ | |
| Experiment 9 | ○ | ○ | ○ | ○ | | | |
| Experiment 10 | ○ | ○ | ○ | | | ○ | |

**[Table 2-1]**

| No | Decomposition target | Metal oxide Metal hydroxide | Solvent | Acid/Base | Product | | | |
|---|---|---|---|---|---|---|---|---|
| | | | | | 1-propanol | 2-propanol | Acetoin | Hydroxyacetone |
| Experiment 1 | Glucose | Lead oxide | Ultrapure water | | | | | |
| Experiment 2 | Glucose | Lead oxide | Ultrapure water | | | | | |
| Experiment 3 | Glucose | Tin oxide | Industrial purified water | | ○ | ○ | ○ | ○ |
| Experiment 4 | Glucose | Germanium oxide | Industrial purified water | | ○ | ○ | | ○ |
| Experiment 5 | Glucose | Silicon oxide | Industrial purified water | | | | ○ | ○ |
| Experiment 6 | Glucose | Alumina | Industrial purified water | | | | ○ | ○ |
| Experiment 7 | Glucose | Anatase type titanium oxide | Industrial purified water | | | | ○ | ○ |
| Experiment 8 | Glucose | Rutile type titanium oxide | Industrial purified water | | | | ○ | ○ |
| Experiment 9 | Glucose | Vanadium pentoxide | Ultrapure water | | | | | |
| Experiment 10 | Glucose | Vanadium pentoxide | Ultrapure water | Sulfuric acid | | | | |

**[Table 2-2]**

| No | Product | | | | | | |
|---|---|---|---|---|---|---|---|
| | Levulinic acid | Furfural | 5-hydroxymethylfurfural | Dihydroxyacetone | 2-hydroxy-γ-butyrolactone | 2-methyl-2-cyclopentene | Propionic acid |
| Experiment 1 | | | | | | | |
| Experiment 2 | | | | | | | |
| Experiment 3 | ○ | ○ | ○ | | | | |
| Experiment 4 | | ○ | ○ | | | | |
| Experiment 5 | | ○ | ○ | | | | |
| Experiment 6 | ○ | ○ | ○ | ○ | ○ | | |
| Experiment 7 | | ○ | ○ | | | | |
| Experiment 8 | ○ | ○ | ○ | | | | |
| Experiment 9 | | | | | | | |
| Experiment 10 | | | | | | | |

**[Table 3-1]**

| No | Decomposition target | Metal oxide Metal hydroxide | Solvent | Acid/Base | Product | | |
|---|---|---|---|---|---|---|---|
| | | | | | Hydrogen | Carbon monoxide | Methane |
| Experiment 11 | Glucose | Vanadium pentoxide | Ultrapure water | Sodium hydroxide | ○ | ○ | |
| Experiment 12 | Glucose | Manganese dioxide | Ultrapure water | | ○ | ○ | |
| Experiment 13 | Glucose | Manganese dioxide | Ultrapure water | Sulfuric acid | ○ | ○ | |
| Experiment 14 | Glucose | Manganese dioxide | Ultrapure water | Sodium hydroxide | ○ | ○ | |
| Experiment 15 | Glucose | α-iron hydroxide | Ultrapure water | | ○ | ○ | ○ |
| Experiment 16 | Glucose | Zinc oxide | Ultrapure water | | ○ | ○ | |
| Experiment 17 | Glucose | Zeolite (with low Si) | Ultrapure water | | ○ | ○ | |
| Experiment 18 | Glucose | Zeolite (with low Si) | Ultrapure water | | ○ | ○ | |
| Experiment 19 | Glucose | Zeolite (with high Si) | Ultrapure water | | ○ | ○ | |
| Experiment 20 | Glucose | Lead oxide zeolite (with high Si) | Ultrapure water | | ○ | ○ | |

**[Table 3-2]**

| No | Product | | | | | | |
|---|---|---|---|---|---|---|---|
| | Acetic acid | Formic acid | Ethylene glycol | Diethylene glycol | Acetone | Methanol | Ethanol |
| Experiment 11 | ○ | ○ | | | | ○ | |
| Experiment 12 | ○ | | | | ○ | ○ | ○ |
| Experiment 13 | ○ | ○ | | | | ○ | |
| Experiment 14 | ○ | ○ | | | | ○ | |
| Experiment 15 | ○ | ○ | ○ | | ○ | ○ | ○ |
| Experiment 16 | ○ | ○ | ○ | | ○ | ○ | |
| Experiment 17 | ○ | ○ | ○ | ○ | ○ | ○ | ○ |
| Experiment 18 | ○ | ○ | ○ | ○ | ○ | ○ | ○ |
| Experiment 19 | ○ | ○ | ○ | ○ | ○ | ○ | ○ |
| Experiment 20 | ○ | ○ | ○ | ○ | ○ | ○ | ○ |

**[Table 4-1]**

| No | Decomposition target | Metal oxide Metal hydroxide | Solvent | Acid/Base | Product | | | |
|---|---|---|---|---|---|---|---|---|
| | | | | | 1-propanol | 2-propanol | Acetoin | Hydroxyacetone |
| Experiment 11 | Glucose | Vanadium pentoxide | Ultrapure water | Sodium hydroxide | | | | |
| Experiment 12 | Glucose | Manganese dioxide | Ultrapure water | | | | | |
| Experiment 13 | Glucose | Manganese dioxide | Ultrapure water | Sulfuric acid | | | | |
| Experiment 14 | Glucose | Manganese dioxide | Ultrapure water | Sodium hydroxide | | | | |
| Experiment 15 | Glucose | α-iron hydroxide | Ultrapure water | | | | | ○ |
| Experiment 16 | Glucose | Zinc oxide | Ultrapure water | | | | ○ | ○ |
| Experiment 17 | Glucose | Zeolite (with low Si) | Ultrapure water | | | | | |
| Experiment 18 | Glucose | Zeolite (with low Si) | Ultrapure water | | | | | |
| Experiment 19 | Glucose | Zeolite (with high Si) | Ultrapure water | | | | | |
| Experiment 20 | Glucose | Lead oxide zeolite (with high Si) | Ultrapure water | | | | | |

**[Table 4-2]**

| No | Product | | | | | | |
|---|---|---|---|---|---|---|---|
| | Levulinic acid | Furfural | 5-hydroxymethylfurfural | Dihydroxyacetone | 2-hydroxy-γ-butyrolactone | 2-methyl-2-cyclopentene | Propionic acid |
| Experiment 11 | | | | | | | |
| Experiment 12 | | | | | | | |
| Experiment 13 | | | | | | | |
| Experiment 14 | | | | | | | |
| Experiment 15 | | | | | | ○ | ○ |
| Experiment 16 | | | | | | | |
| Experiment 17 | | | | | | | |
| Experiment 18 | | | | | | | |
| Experiment 19 | | | | | | | |
| Experiment 20 | | | | | | | |

### [Experiment 21]

In Experiment 21, 3.1376 g (0.019 mol) in weight of wheat derived starch (approximately 162 g/mol, manufactured by FUJIFILM Wako Pure Chemical Corporation), 27.5035 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of starch is 0.387 mol/L. In this state, a decomposition reaction was carried out at 200 °C for 1.5 hours while stirring at a rotation speed of 300 rpm. As a result, from the gas phase, 0.0086 mol (44.1 mol%) of hydrogen and 0.00020 mol (1.0 mol%) of carbon monoxide were obtained, and from the liquid phase, as aqueous solutions, 0.0086 mol/L (2.2 mol%) of acetone, 0.015 mol/L (3.9 mol%) of methanol, 0.0050 mol/L (1.3 mol%) of ethanol, 0.07 mol/L (18.0 mol%) of acetic acid, and 0.089 mol/L (23 mol%) of formic acid were obtained.

### [Experiment 22]

In Experiment 22, 3.0419 g (0.019 mol) in weight of insoluble dietary fiber cellulose (approximately 162 g/mol, manufactured by Healthy Company Co., Ltd.), 27.2588 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (MMJ-100 manufactured by OMLABO TECH CO., LTD., capacity 120 mL), and the container was sealed. At this point, the concentration of cellulose is 0.376 mol/L. In this state, a decomposition reaction was carried out at 200 °C for 1.5 hours while stirring at a rotation speed of 400 rpm. As a result, from the gas phase, 0.0016 mol (6.2 mol%) of hydrogen was obtained, and from the liquid phase, as aqueous solutions, 0.0028 mol/L (0.75 mol%) of acetone, 0.027 mol/L (7.2 mol%) of methanol, 0.0035 mol/L (0.94 mol%) of ethanol, 0.046 mol/L (12.1 mol%) of acetic acid, and 0.069 mol/L (18.5 mol%) of formic acid were obtained.

According to Experiments 21 and 22, starch (α-1,4-linked glucose polymer) and cellulose (β-1,4-linked glucose polymer) can be decomposed by the action of lead oxide, and industrially useful chemical components such as a combustible gas, an alcohol, an organic acid, and a ketone can be easily obtained. The results of Experiments 21 and 22 are considered to have been brought about by the fact that the standard oxidation-reduction potential of lead oxide is higher than that of water, whereby water is spontaneously oxidized on its surface, generating protons (H⁺), electrons (e⁻), and oxygen (O₂), thus creating a strong acidic environment. Therefore, results similar to those of Experiments 21 and 22 can be obtained by using metal oxides and metal hydroxides that have a higher standard oxidation-reduction potential than that of water, other than lead oxide (the same applies to other examples).

It is well known that coffee beans are a trade product that is traded in large quantities next to oil, and the extraction residue of coffee beans is a waste that is generated on a global scale. The main components of the extraction residue of coffee beans are galactomannan, a copolymer (= polysaccharide) of galactose (a hexose) and mannose (a hexose), and arabinogalactan, a polysaccharide composed of arabinose (a pentose) and galactose (a hexose) (see Patent Literature 2). In the following Experiments 23 to 26, galactose, mannose, and arabinose, which are monosaccharides similar to glucose, and the extraction residue of coffee beans were each decomposed by lead oxide.

### [Experiment 23]

In Experiment 23, 3.0268 g (0.017 mol) in weight of galactose (approximately 180 g/mol, manufactured by KISHIDA CHEMICAL CO., LTD.), 27.2608 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of galactose is 0.3360 mol/L. In this state, a decomposition reaction was carried out at 200 °C for 1.5 hours while stirring at a rotation speed of 200 rpm. As a result, from the gas phase, 0.002 mol (11.7 mol%) of hydrogen was obtained, and from the liquid phase, as aqueous solutions, 0.0083 mol/L (2.5 mol%) of acetone, 0.0019 mol/L (0.57 mol%) of methanol, 0.0008 mol/L (0.24 mol%) of ethanol, 0.026 mol/L (7.7 mol%) of acetic acid, 0.019 mol/L (5.5 mol%) of formic acid, 0.0037 mol/L (1.1 mol%) of ethylene glycol, and 0.066 mol/L (1.9 mol%) of diethylene glycol were obtained. And a clear peak of acetoin was observed in GCMS.

### [Experiment 24]

In Experiment 24, 1.0779 g (0.006 mol) in weight of mannose (approximately 180 g/mol, manufactured by KISHIDA CHEMICAL CO., LTD.), 9.0896 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of mannose is 0.12 mol/L. In this state, a decomposition reaction was carried out at 200 °C for 1 hour while stirring at a rotation speed of 200 rpm. As a result, from the gas phase, 0.0028 mol (48.3 mol%) of hydrogen and 0.00045 mol (0.76 mol%) of carbon monoxide were obtained, and from the liquid phase, as aqueous solutions, 0.0044 mol/L (3.7 mol%) of acetone, 0.0018 mol/L (1.5 mol%) of methanol, 0.002 mol/L (1.6 mol%) of ethanol, 0.0017 mol/L (1.5 mol%) of acetic acid, 0.002 mol/L (1.7 mol%) of formic acid, and 0.0016 mol/L (1.4 mol%) of ethylene glycol were obtained.

### [Experiment 25]

In Experiment 25, 1.0160 g (0.0068 mol) in weight of arabinose (approximately 150 g/mol, manufactured by FUJIFILM Wako Pure Chemical Corporation), 9.0788 g of lead oxide (PbO₂, manufactured by KANTO CHEMICAL CO., INC.), and 50 mL of industrial purified water were placed in a pressure-resistant container (TEM-D100M manufactured by Taiatsu Techno Corporation, capacity 128 mL), and the container was sealed. At this point, the concentration of arabinose is 0.14 mol/L. In this state, a decomposition reaction was carried out at 200 °C for 1 hour while stirring at a rotation speed of 200 rpm. As a result, from the gas phase, 0.002 mol (29 mol%) of hydrogen and 0.00003 mol (0.4 mol%) of carbon monoxide were obtained, and from the liquid phase, as aqueous solutions, 0.0011 mol/L (0.82 mol%) of acetone, 0.00026 mol/L (2.3 mol%) of methanol, 0.017 mol/L (12.6 mol%) of acetic acid, and 0.017 mol/L (12.6 mol%) of formic acid were obtained.

### [Experiment 26]

In Experiment 26, a dry weight of 10 g (0.056 mol) of the extraction residue of coffee beans (medium roast fine grind regarded as 180 g/mol), a commercially available paper coffee filter (cellulose, dry weight 1 g), 90.8464 g of lead oxide (PbO₂, manufactured by KANTO CHEMICAL CO., INC.), and 300 mL of ultrapure water (specific resistance 18.2 MΩ·cm, manufactured by ELGA) were placed in a pressure-resistant container (TEM-D1000M manufactured by Taiatsu Techno Corporation, capacity 1550 mL), and the container was sealed. At this point, the concentration of the extraction residue of coffee beans is 0.2 mol/L when all the coffee beans are considered to be dissolved. In this state, a decomposition reaction was carried out at 200 °C for 2.5 hours while stirring at a rotation speed of 200 rpm. As a result, from the gas phase, 0.030 mol (48.9 mol%) of hydrogen and 0.0018 mol (2.9 mol%) of carbon monoxide were obtained, and from the liquid phase, as aqueous solutions, 0.0013 mol/L (0.64 mol%) of acetone, 0.0046 mol/L (2.3 mol%) of methanol, 0.0012 mol/L (0.59 mol%) of ethanol, 0.028 mol/L (13.5 mol%) of acetic acid, and 0.023 mol/L (11.4 mol%) of formic acid were obtained.

According to Experiments 23 to 26, it is possible to easily obtain industrially useful chemical components such as a combustible gas, an alcohol, an organic acid, and a ketone by effectively decomposing monosaccharides other than glucose and their polymers, polysaccharides, with a metal oxide or a metal hydroxide including lead oxide. Further, according to Experiment 26, it can be seen that the extraction residue of coffee beans itself can also be a resource.

### [Experiment 27]

In Experiment 27, 3.0378 g (0.019 mol) in weight of an unused paper coffee filter (cellulose, 3 sheets regarded as having a molecular weight of 180 g/mol), 27.2352 g of lead oxide (PbO₂, manufactured by KANTO CHEMICAL CO., INC.), 0.0974 g of sodium hydroxide (0.002435 mol, manufactured by KISHIDA CHEMICAL CO., LTD.), and 50 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of the paper coffee filter is 0.375 mol/L when all the filters are considered to be dissolved, and the pH is 13. In this state, the decomposition reaction was carried out at 200 °C for 4 hours while stirring at a rotation speed of 200 rpm. As a result, from the gas phase, 0.003 mol (16.1 mol%) of hydrogen and 0.0001 mol (0.54 mol%) of carbon monoxide were obtained, and from the liquid phase, as aqueous solutions, 0.0019 mol/L (0.5 mol%) of acetone, 0.005 mol/L (1.4 mol%) of methanol, 0.00026 mol/L (0.07 mol%) of ethanol, 0.0063 mol/L (1.7 mol%) of acetic acid, 0.0038 mol/L (1.0 mol%) of formic acid, 0.0020 mol/L (0.56 mol%) of ethylene glycol, and 0.0019 mol/L (0.5 mol%) of diethylene glycol were obtained.

According to Experiment 27, it is possible to easily obtain industrially useful chemical components such as a combustible gas, an alcohol, an organic acid, and a ketone by decomposing a paper filter (cellulose) for coffee dripping with a metal oxide or a metal hydroxide including lead oxide. Although the results of the decomposition experiment on insoluble powdered cellulose are already shown in Experiment 22, it can be seen that even for the same cellulose, the yield of decomposition products differs between the form of paper and the form of powder.

### [Experiment 28]

In Experiment 28, 3.0426 g (0.017 mol) in weight of fructose (approximately 180 g/mol, manufactured by KISHIDA CHEMICAL CO., LTD.), 27.0261 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of fructose is 0.338 mol/L. In this state, a decomposition reaction was carried out at 200 °C for 1.5 hours while stirring at a rotation speed of 200 rpm. As a result, from the gas phase, 0.002 mol (13.2 mol%) of hydrogen and 0.0001 mol (0.60 mol%) of carbon monoxide were obtained, and from the liquid phase, as aqueous solutions, 0.00096 mol/L (0.28 mol%) of acetone, 0.0024 mol/L (0.07 mol%) of methanol, 0.00071 mol/L (0.21 mol%) of ethanol, 0.034 mol/L (10.1 mol%) of acetic acid, 0.0092 mol/L (2.7 mol%) of formic acid, 0.0020 mol/L (0.56 mol%) of ethylene glycol, and 0.00034 mol/L (0.001 mol%) of diethylene glycol were obtained.

### [Experiment 29]

In Experiment 29, 10.1350 g (0.03 mol) in weight of sucrose (approximately 342 g/mol, manufactured by NACALAI TESQUE, INC.), 91.6667 g of lead oxide (PbO₂, manufactured by KANTO CHEMICAL CO., INC.), and 300 mL of ultrapure water (specific resistance 18.2 MΩ·cm, manufactured by ELGA) were placed in a pressure-resistant container (TEM-D1000M manufactured by Taiatsu Techno Corporation, capacity 1550 mL), and the container was sealed. At this point, the concentration of sucrose is 0.099 mol/L. In this state, a decomposition reaction was carried out at 200 °C for 1.5 hours while stirring at a rotation speed of 200 rpm. As a result, from the gas phase, 0.031 mol (105.27 mol%) of hydrogen and 0.0026 mol (8.67 mol%) of carbon monoxide were obtained, and from the liquid phase, as aqueous solutions, 0.0057 mol/L (5.8 mol%) of acetone, 0.015 mol/L (14.9 mol%) of methanol, 0.016 mol/L (16.6 mol%) of ethanol, 0.039 mol/L (39.3 mol%) of acetic acid, 0.00073 mol/L (0.74 mol%) of formic acid, and 0.00035 mol/L (0.35 mol%) of ethylene glycol were obtained.

Fructose is an important biologically derived monosaccharide. It forms a disaccharide with glucose and becomes sucrose (saccharose, sugar). A large amount of sucrose remains in leftover waste (food waste). According to Experiments 28 and 29, it is possible to easily obtain industrially useful chemical components such as a combustible gas, an alcohol, an organic acid, and a ketone by decomposing fructose and sucrose with a metal oxide or a metal hydroxide including lead oxide.

### [Experiment 30]

In Experiment 30, 3.0044 g (0.0083 mol) in weight of maltose monohydrate (approximately 360 g/mol, manufactured by KISHIDA CHEMICAL CO., LTD.), 29.4174 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of maltose monohydrate is 0.17 mol/L. In this state, a decomposition reaction was carried out at 200 °C for 1 hour and 35 minutes while stirring at a rotation speed of 300 rpm. As a result, from the gas phase, 0.012 mol (143.8 mol%) of hydrogen and 0.00015 mol (1.8 mol%) of carbon monoxide were obtained, and from the liquid phase, as aqueous solutions, 0.0046 mol/L (2.7 mol%) of acetone, 0.0021 mol/L (1.2 mol%) of methanol, 0.0011 mol/L (0.67 mol%) of ethanol, 0.023 mol/L (13.5 mol%) of acetic acid, 0.013 mol/L (7.9 mol%) of formic acid, 0.00068 mol/L (0.041 mol%) of ethylene glycol, and 0.00088 mol/L (0.053 mol%) of diethylene glycol were obtained.

### [Experiment 31]

In Experiment 31, 3.0774 g (0.0083 mol) in weight of lactose monohydrate (approximately 360 g/mol, manufactured by KISHIDA CHEMICAL CO., LTD.), 27.5832 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (manufactured by OMLABO TECH CO., LTD., capacity 120 mL), and the container was sealed. At this point, the concentration of lactose monohydrate is 0.17 mol/L. In this state, a decomposition reaction was carried out at 200 °C for 1 hour and 35 minutes while stirring at a rotation speed of 450 rpm. As a result, from the gas phase, 0.0044 mol (51.3 mol%) of hydrogen and 0.00015 mol (1.8 mol%) of carbon monoxide were obtained, and from the liquid phase, as aqueous solutions, 0.0059 mol/L (3.5 mol%) of acetone, 0.0014 mol/L (0.81 mol%) of methanol, 0.0053 mol/L (3.1 mol%) of ethanol, 0.029 mol/L (16.7 mol%) of acetic acid, 0.014 mol/L (8.3 mol%) of formic acid, 0.0019 mol/L (1.1 mol%) of ethylene glycol, and 0.0029 mol/L (1.7 mol%) of diethylene glycol were obtained.

Maltose and lactose are important disaccharides that humans eat, and are present in large amounts in waste. Maltose is contained in large quantities in barley, and has the same structure as a linear component of starch, consisting of two glucoses linked together, while lactose is a disaccharide of glucose and galactose. According to Experiment 30, similarly to starch and glucose, maltose can also be effectively decomposed with a metal oxide or a metal hydroxide including lead oxide, and as a result, industrially useful chemical components such as a combustible gas, an alcohol, an organic acid, and a ketone can be easily obtained. Further, according to Experiment 31, similarly to glucose and galactose, lactose can also be decomposed with a metal oxide or a metal hydroxide including lead oxide, and as a result, industrially useful chemical components such as a combustible gas, an alcohol, an organic acid, and a ketone can be easily obtained.

### [Experiment 32]

In Experiment 32, 3.0710 g (0.009 mol) in weight of cellobiose (approximately 342 g/mol, manufactured by FUJIFILM Wako Pure Chemical Corporation), 27.2601 g of lead oxide (PbO₂, manufactured by KANTO CHEMICAL CO., INC.), and 300 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of cellobiose is 0.18 mol/L. In this state, a decomposition reaction was carried out at 200 °C for 1 hour and 50 minutes while stirring at a rotation speed of 200 rpm. As a result, from the gas phase, 0.002 mol (23.1 mol%) of hydrogen and 0.0013 mol (14.9 mol%) of carbon monoxide were obtained, and from the liquid phase, as aqueous solutions, 0.0063 mol/L (3.5 mol%) of acetone, 0.0045 mol/L (2.5 mol%) of methanol, 0.0011 mol/L (0.63 mol%) of ethanol, 0.054 mol/L (30.2 mol%) of acetic acid, 0.04 mol/L (20.6 mol%) of formic acid, and 0.00045 mol/L (0.25 mol%) of ethylene glycol were obtained.

Cellobiose is a disaccharide obtained by decomposition of cellulose. According to Experiment 32, cellobiose can be effectively decomposed with a metal oxide or a metal hydroxide including lead oxide, and as a result, industrially useful chemical components such as a combustible gas, an alcohol, an organic acid, and a ketone can be easily obtained.

### [Experiment 33]

In Experiment 33, 10.0037 g (0.052 mol) in weight of citrus pectin (approximately 194 g/mol, manufactured by FUJIFILM Wako Pure Chemical Corporation), 90.5170 g of lead oxide (PbO₂, manufactured by KANTO CHEMICAL CO., INC.) and 300 mL of ultrapure water (specific resistance 18.2 MΩ·cm, manufactured by ELGA) were placed in a pressure-resistant container (TEM-D1000M manufactured by Taiatsu Techno Corporation, capacity 1550 mL), and the container was sealed. At this point, the concentration of pectin is 0.17 mol/L. In this state, the decomposition reaction was carried out at 200 °C for 2 hours while stirring at a rotation speed of 200 rpm. As a result, from the gas phase, 0.025 mol (48.5 mol%) of hydrogen and 0.0015 mol (2.9 mol%) of carbon monoxide were obtained, and from the liquid phase, as aqueous solutions, 0.0043 mol/L (2.5 mol%) of acetone, 0.096 mol/L (56.1 mol%) of methanol, 0.0058 mol/L (3.4 mol%) of ethanol, 0.022 mol/L (12.7 mol%) of acetic acid, and 0.01 mol/L (5.7 mol%) of formic acid were obtained.

Pectin is one of representative complex polysaccharides. Pectin is always contained in cell walls of plants, and resides between cellulose fibers to stabilize the morphology of plant cells. Pectin is particularly abundant in fruits and is also used as a thickener for foods such as jams. Therefore, pectin is a polyol contained in large quantities not only in household food waste but also in industrial waste and sludge from food factories. According to Experiment 33, it is possible to easily obtain industrially useful chemical components such as a combustible gas, an alcohol, an organic acid, and a ketone by decomposing pectin with a metal oxide or a metal hydroxide including lead oxide.

### [Experiment 34]

In Experiment 34, 3.0241 g (0.017 mol) in weight of dealkalized lignin (approximately 182 g/mol, manufactured by Tokyo Chemical Industry Co., Ltd.), 27.2113 g of lead oxide (PbO₂, manufactured by KANTO CHEMICAL CO., INC.) and 50 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of lignin is 0.33 mol/L. In this state, a decomposition reaction was carried out at 200 °C for 3 hours while stirring at a rotation speed of 200 rpm. As a result, from the gas phase, 0.016 mol (9.4 mol%) of hydrogen and 0.000076 mol (0.45 mol%) of carbon monoxide were obtained, and from the liquid phase, as aqueous solutions, 0.0058 mol/L (1.7 mol%) of acetone, 0.089 mol/L (26.6 mol%) of methanol, 0.00098 mol/L (0.29 mol%) of ethanol, and 0.020 mol/L (6.1 mol%) of acetic acid were obtained.

Although a molecular weight of lignin in wood is said to be thousands to tens of thousands, a molecular structure of the hydrolyzed minimum unit was considered to be phenol-glycerol. In other words, an ideal form was considered to be HO<C6H4>C*(OH)CH(OH)C*H(OH) = 182 g/mol (<C6H4>: benzene nucleus, *: crosslinking point) (see Patent Literature 3).

### [Experiment 35]

In Experiment 35, 10.2407 g (0.06 mol) in weight of cypress powder (172 g/mol (the values of the molecular weight 162 of cellulose and the molecular weight 182 of lignin were taken as the molar mass)), 90.8575 g of lead oxide (PbO₂, manufactured by KANTO CHEMICAL CO., INC.), and 300 mL of ultrapure water (specific resistance 18.2 MΩ·cm, manufactured by ELGA) were placed in a pressure-resistant container (TEM-D1000M manufactured by Taiatsu Techno Corporation, capacity 1550 mL), and the container was sealed. At this point, the concentration of the wood powder is 0.2 mol/L assuming that all the wood powder is dissolved. In this state, the decomposition reaction was carried out at 200 °C for 4 hours while stirring at a rotation speed of 200 rpm. As a result, from the gas phase, 0.02 mol (33.5 mol%) of hydrogen and 0.0015 mol (2.6 mol%) of carbon monoxide were obtained, and from the liquid phase, as aqueous solutions, 0.00019 mol/L (0.09 mol%) of acetone, 0.031 mol/L (15.6 mol%) of methanol, 0.011 mol/L (0.55 mol%) of ethanol, and 0.016 mol/L (7.8 mol%) of acetic acid were obtained.

### [Experiment 36]

In Experiment 36, 3.0685 g (0.02 mol) in weight of xylan ([C₅H₈O₄]ₙ, C₅H₈O₄ = 132 g/mol, main component of hemicellulose, manufactured by Tokyo Chemical Industry Co., Ltd.), 27.3635 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (MMJ-100 manufactured by OMLABO TECH CO., LTD., capacity 120 mL), and the container was sealed. At this point, the concentration of xylan is 0.4 mol/L. In this state, a decomposition reaction was carried out at 200 °C for 1 hour while stirring at a rotation speed of 350 rpm. As a result, from the gas phase, 0.0049 mol (23.8 mol%) of hydrogen and 0.00010 mol (0.49 mol%) of carbon monoxide were obtained, and from the liquid phase, as aqueous solutions, 0.0046 mol/L (1.1 mol%) of acetone, 0.0016 mol/L (0.28 mol%) of methanol, 0.0057 mol/L (1.4 mol%) of ethanol, 0.039 mol/L (9.6 mol%) of acetic acid, 0.0058 mol/L (1.4 mol%) of formic acid, 0.015 mol/L (3.8 mol%) of ethylene glycol, and 0.00045 mol/L (0.11 mol%) of diethylene glycol were obtained.

### [Experiment 37]

In Experiment 37, 3.0478 g (0.02 mol) in weight of xylose (a monosaccharide constituting xylan, approximately 150 g/mol, manufactured by Tokyo Chemical Industry Co., Ltd.), 27.3635 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of xylose is 0.4 mol/L. In this state, a decomposition reaction was carried out at 200 °C for 1.5 hours while stirring at a rotation speed of 350 rpm. As a result, from the gas phase, 0.0026 mol (12.9 mol%) of hydrogen and 0.00008 mol (0.38 mol%) of carbon monoxide were obtained, and from the liquid phase, as aqueous solutions, 0.0082 mol/L (2.0 mol%) of acetone, 0.0021 mol/L (0.52 mol%) of methanol, 0.000082 mol/L (0.20 mol%) of ethanol, 0.027 mol/L (6.7 mol%) of acetic acid, and 0.0093 mol/L (2.3 mol%) of formic acid were obtained.

According to Experiment 34, it is possible to easily obtain industrially useful chemical components such as a combustible gas, an alcohol, an organic acid, and a ketone by decomposing lignin, which is a polyol obtained from wood, with a metal oxide or a metal hydroxide including lead oxide. Further, according to Experiment 35, it is possible to easily obtain industrially useful chemical components such as a combustible gas, an alcohol, an organic acid, and a ketone by decomposing wood itself with a metal oxide or a metal hydroxide including lead oxide.

Hemicellulose is also an important polyol in wood. According to Experiment 36, it is possible to easily obtain industrially useful chemical components such as a combustible gas, an alcohol, an organic acid, and a ketone by decomposing xylan as a main component of hemicellulose with a metal oxide or a metal hydroxide including lead oxide. Considering that hemicellulose is a mixture containing various saccharides with xylose as the main component, it can be said that hemicellulose can be decomposed by applying the method according to Experiment 36. Further, according to Experiment 37, it is possible to easily obtain industrially useful chemical components such as a combustible gas, an alcohol, an organic acid, and a ketone by decomposing xylose, the minimum unit (monosaccharide) of xylan, with a metal oxide or a metal hydroxide including lead oxide.

By applying Experiments 34 to 37, there is a possibility that wood-based waste originating from forests can be converted into industrial resources.

### [Experiment 38]

In Experiment 38, 3.0832 g (0.019 mol) in weight of domestic sludge that was collected from a wastewater treatment plant and formed into a cake shape by a screw type dewatering device (162 g/mol, using the molecular weights of starch and cellulose), 27.2927 g of lead oxide (PbO₂, manufactured by KANTO CHEMICAL CO., INC.), and 50 mL of industrial purified water were placed in a pressure-resistant container (MMJ-100 manufactured by OMLABO TECH CO., LTD., capacity 120 mL), and the container was sealed. At this point, the concentration of the domestic sludge is 0.38 mol/L assuming that all the sludge is dissolved. In this state, a decomposition reaction was carried out at 200 °C for 1.5 hours while stirring at a rotation speed of 430 rpm. As a result, from the gas phase, 0.00093 mol (4.9 mol%) of hydrogen and 0.0001 mol (0.54 mol%) of carbon monoxide were obtained, and from the liquid phase, as aqueous solutions, 0.0013 mol/L (0.33 mol%) of acetone, 0.0018 mol/L (0.47 mol%) of methanol, 0.00056 mol/L (0.15 mol%) of ethanol, 0.0079 mol/L (2.1 mol%) of acetic acid, 0.0063 mol/L (1.6 mol%) of formic acid, 0.00045 mol/L (0.12 mol%) of ethylene glycol, and 0.0012 mol/L (0.31 mol%) of diethylene glycol were obtained.

### [Experiment 39]

In Experiment 39, 3.0719 g (0.0095 mol of polyol component) in weight of dried papermaking sludge in a ball shape with a diameter of approximately 10 mm to 20 mm collected from a paper mill (The polyol component was 162 g/mol based on cellulose. 1/2 of the weighed sample is the polyol component, and the remaining 1/2 is inorganic salts such as alumina, silica, or calcium oxide. See Non Patent Literature 1.), 27.2927 g of lead oxide (PbO₂, manufactured by KANTO CHEMICAL CO., INC.), and 50 mL of industrial purified water were placed in a pressure-resistant container (TEM-D100M manufactured by Taiatsu Techno Corporation, capacity 128 mL), and the container was sealed. At this point, the concentration of the papermaking sludge is 0.19 mol/L assuming that all the sludge is dissolved. In this state, a decomposition reaction was started at a set temperature of 200 °C while stirring at a rotation speed of 250 rpm. Even when the temperature of the reaction container reached 200 °C, the temperature inside the container continued to rise and reached 280 °C after 10 minutes. At this point, a safety device of a temperature control device was activated and a heater was stopped. Thereafter, the mixture was naturally cooled as it was. Therefore, the reaction time is 10 minutes, from 200 °C to 280 °C. As a result, from the gas phase, 0.00093 mol (10.8 mol%) of hydrogen and 0.000051 mol (0.54 mol%) of carbon monoxide were obtained, and from the liquid phase, as aqueous solutions, 0.00017 mol/L (0.09 mol%) of acetone, 0.0016 mol/L (0.84 mol%) of methanol, 0.00023 mol/L (0.12 mol%) of ethanol, 0.0073 mol/L (3.8 mol%) of acetic acid, 0.0061 mol/L (3.2 mol%) of formic acid, 0.00048 mol/L (0.26 mol%) of ethylene glycol, and 0.0012 mol/L (0.62 mol%) of diethylene glycol were obtained.

According to Experiments 38 and 39, it is possible to easily obtain industrially useful chemical components such as a combustible gas, an alcohol, an organic acid, and a ketone by decomposing actual domestic sludge and papermaking sludge with a metal oxide or a metal hydroxide including lead oxide.

### [Experiment 40]

In Experiment 40, 3.0194 g (0.033 mol) in weight of glycerol (approximately 92 g/mol, SIGMA-ALDRICH), 27.2749 g of lead oxide (PbO₂, manufactured by KANTO CHEMICAL CO., INC.), and 50 mL of ultrapure water (specific resistance 18.2 MΩ·cm, manufactured by ELGA) were placed in a pressure-resistant container (TEM-D100M manufactured by Taiatsu Techno Corporation, capacity 128 mL), and the container was sealed. At this point, the concentration of glycerol is 0.66 mol/L. In this state, a reaction was carried out at 200 °C for 1 hour while stirring at a rotation speed of 200 rpm. As a result, from the gas phase, 0.013 mol (38.5 mol%) of hydrogen and 0.000092 mol (0.28 mol%) of carbon monoxide were obtained, and from the liquid phase, as aqueous solutions, 0.00039 mol/L (0.06 mol%) of acetone, 0.039 mol/L (6 mol%) of methanol, 0.0044 mol/L (0.67 mol%) of ethanol, 0.011 mol/L (17.4 mol%) of acetic acid, 0.0048 mol/L (0.73 mol%) of formic acid, and 0.0019 mol/L (2.9 mol%) of ethylene glycol were obtained.

### [Experiment 41]

In Experiment 41, 10.2482 g (0.047 mol) in weight of triacetin (218 g/mol, manufactured by Hayashi Pure Chemical Ind., Ltd.), 90.2412 g of lead oxide (PbO₂, manufactured by KANTO CHEMICAL CO., INC.), and 300 mL of ultrapure water (specific resistance 18.2 MΩ·cm, manufactured by ELGA) were placed in a pressure-resistant container (TEM-D100M manufactured by Taiatsu Techno Corporation 128 mL), and the container was sealed. At this point, the concentration of triacetin is 0.16 mol/L. In this state, a reaction was carried out at 200 °C for 1 hour and 40 minutes while stirring at a rotation speed of 200 rpm. As a result, from the gas phase, 0.015 mol (32.0 mol%) of hydrogen and 0.0010 mol (2.2 mol%) of carbon monoxide were obtained, and from the liquid phase, as aqueous solutions, 0.00041 mol/L (0.26 mol%) of acetone, 0.017 mol/L (11.1 mol%) of methanol, 0.0023 mol/L (1.5 mol%) of ethanol, 0.14 mol/L (90.4 mol%) of acetic acid, 0.077 mol/L (49.6 mol%) of formic acid, and 0.00036 mol/L (0.23 mol%) of ethylene glycol were obtained.

### [Experiment 42]

In Experiment 42, 1.0420 g (0.0011 mol) in weight of soybean oil (approximately 917 g/mol (Non Patent Literature 2), manufactured by FUJIFILM Wako Pure Chemical Corporation), 9.0995 g of lead oxide (PbO₂, manufactured by KANTO CHEMICAL CO., INC.), and 50 mL of industrial purified water were placed in a pressure-resistant container (TEM-D100M manufactured by Taiatsu Techno Corporation, capacity 128 mL), and the container was sealed. At this point, the concentration of soybean oil is 0.023 mol/L. In this state, a reaction was carried out at 200 °C for 1.5 hours while stirring at a rotation speed of 200 rpm. As a result, from the gas phase, 0.0017 mol (146.4 mol%) of hydrogen, 0.000050 mol (4.4 mol%) of carbon monoxide, and 0.00020 mol (17.7 mol%) of methane were obtained, and from the liquid phase, as aqueous solutions, 0.0018 mol/L (7.7 mol%) of acetone, 0.0014 mol/L (6.3 mol%) of methanol, 0.00029 mol/L (1.3 mol%) of ethanol, 0.0049 mol/L (21.7 mol%) of acetic acid, 0.0018 mol/L (8.1 mol%) of formic acid, 0.00031 mol/L (1.4 mol%) of ethylene glycol, 0.0004 mol/L (1.7 mol%) of 1-butanol, and 0.0005 mol/L (2.2 mol%) of hydroxyacetone were obtained. Further, the water-insoluble components attached to lead oxide were quantified by drying the lead oxide powder separated by filtration at 80 °C for 7 hours, adding 50 mL of hexane to extract the water-insoluble components, and measuring the hexane solution with GCMS. The GCMS apparatus, column, helium flow rate, and measurement temperature are all the same as those for the analysis of the aqueous solution components. As a result, clear peaks of GCMS were obtained for 3-hexanol, 2-hexanol, pentadecane, octadecane, 8-heptadecene, hexanoic acid, heptanoic acid, and octanoic acid. It is clear that these components are derived from fatty acids of soybean oil.

### [Experiment 43]

In Experiment 43, 2.1 g (0.0025 mol) in weight of crude palm oil (CPO: Crude Palm Oil, approximately 850 g/mol (see Non Patent Literatures 3 and 4), produced in Indonesia), 9.02 g of lead oxide (PbO₂, manufactured by KANTO CHEMICAL CO., INC.), 50 mL of ultrapure water (specific resistance 18.2 MΩ ▪ cm, manufactured by ELGA), and 1 mL of 0.1 mol/L sulfuric acid were placed in a pressure-resistant container (MMJ-100 manufactured by OMLABO TECH CO., LTD., capacity 120 mL), and the container was sealed. At this point, the concentration of crude palm oil is 0.049 mol/L. In this state, a reaction was carried out at 200 °C for 2 hours while stirring at a rotation speed of 400 rpm. As a result, from the gas phase, 0.0029 mol (115.8 mol%) of hydrogen, 0.00015 mol (6.1 mol%) of carbon monoxide, and 0.0020 mol (81.3 mol%) of methane were obtained, and from the liquid phase, as aqueous solutions, 0.0018 mol/L (3.7 mol%) of acetone, 0.0029 mol/L (5.9 mol%) of methanol, 0.0019 mol/L (3.4 mol%) of ethanol, 0.0041 mol/L (8.4 mol%) of acetic acid, 0.0065 mol/L (13.5 mol%) of formic acid, 0.0017 mol/L (3.5 mol%) of ethylene glycol, 0.0013 mol/L (2.6 mol%) of diethylene glycol, and 0.066 mol/L (133.2 mol%) of 1-propanol were obtained.

### [Experiment 44]

In Experiment 44, 2.03 g (0.013 mol) in weight of a palm kernel shell (PKS: Palm Kernel Shell, approximately 162 g/mol since it is close to cellulose, produced in Indonesia), 9.02 g of lead oxide (PbO₂, manufactured by KANTO CHEMICAL CO., INC.), 50 mL of ultrapure water (specific resistance 18.2 MΩ ▪ cm, manufactured by ELGA), and 1 mL of 0.1 mol/L sulfuric acid were placed in a pressure-resistant container (MMJ-100 manufactured by OMLABO TECH CO., LTD., capacity 120 mL), and the container was sealed. At this point, the concentration of PKS is 0.25 mol/L assuming that all the PKS is dissolved. In this state, a reaction was carried out at 200 °C for 2 hours while stirring at a rotation speed of 400 rpm. As a result, from the gas phase, 0.0037 mol (29.6 mol%) of hydrogen, 0.00005 mol (0.4 mol%) of carbon monoxide, and 0.0005 mol (4 mol%) of methane were obtained, and from the liquid phase, as aqueous solutions, 0.0026 mol/L (1.0 mol%) of acetone, 0.0066 mol/L (2.6 mol%) of methanol, 0.0027 mol/L (1.1 mol%) of ethanol, 0.0084 mol/L (3.4 mol%) of acetic acid, 0.0021 mol/L (0.85 mol%) of formic acid, 0.0028 mol/L (1.1 mol%) of ethylene glycol, and 0.0016 mol/L (0.63 mol%) of diethylene glycol were obtained.

### [Experiment 45]

In Experiment 45, 2.1 g (0.013 mol) in weight of a sludge component submerged in a palm oil mill effluent that is slightly wet (POME: Palm Oil Mill Effluent, 162 g/mol since it is close to cellulose, produced in Indonesia), 9.02 g of lead oxide (PbO₂, manufactured by KANTO CHEMICAL CO., INC.), 50 mL of ultrapure water (specific resistance 18.2 MΩ▪cm, manufactured by ELGA), and 1 mL of 0.1 mol/L sulfuric acid were placed in a pressure-resistant container (MMJ-100 manufactured by OMLABO TECH CO., LTD., capacity 120 mL), and the container was sealed. At this point, the concentration of the POEM sludge is 0.26 mol/L assuming that all the sludge is dissolved. In this state, a reaction was carried out at 200 °C for 2 hours while stirring at a rotation speed of 400 rpm. As a result, from the gas phase, 0.0010 mol (7.7 mol%) of hydrogen and 0.000050 mol (0.39 mol%) of carbon monoxide were obtained, and from the liquid phase, as aqueous solutions, 0.0017 mol/L (0.64 mol%) of acetone, 0.0030 mol/L (1.2 mol%) of methanol, 0.0018 mol/L (0.71 mol%) of ethanol, 0.0053 mol/L (2.0 mol%) of acetic acid, 0.0015 mol/L (0.59 mol%) of formic acid, 0.0019 mol/L (0.72 mol%) of ethylene glycol, and 0.0018 mol/L (0.68 mol%) of diethylene glycol were obtained.

### [Experiment 46]

In Experiment 46, 50 mL of an aqueous solution of POME from Indonesia (with solid content being 4% of the total (see Non Patent Literature 5), equivalent to 3 g of cellulose, 162 g/mol, 1.5 g/mL, 0.019 mol, and 0.37 mol/L), 9.02 g of lead oxide (PbO₂, manufactured by KANTO CHEMICAL CO., INC.), and 1 mL of 0.1 mol/L sulfuric acid were placed in a pressure-resistant container (MMJ-100 manufactured by OMLABO TECH CO., LTD., capacity 120 mL), and the container was sealed. In this state, a decomposition reaction was carried out at 200 °C for 2 hours while stirring at a rotation speed of 400 rpm. As a result, from the gas phase, 0.072 mol (391.2 mol%) of hydrogen, 0.0012 mol (6.7 mol%) of carbon monoxide, and 0.016 mol (88.9%) of methane were obtained, and from the liquid phase, as aqueous solutions, 0.0049 mol/L (1.3 mol%) of acetone, 0.022 mol/L (6.2 mol%) of methanol, 0.0089 mol/L (2.4 mol%) of ethanol, 0.023 mol/L (6.2 mol%) of acetic acid, 0.0014 mol/L (0.39 mol%) of formic acid, 0.0023 mol/L (0.62 mol%) of ethylene glycol, 0.0018 mol/L (0.48 mol%) of diethylene glycol, and 0.75 mol/L (202.0 mol%) of 1-propanol were obtained.

Oils and fats that are oil components derived from plants and animals, such as edible oils (vegetable oils) and lard after cooking, are now discarded as waste. A lipid is a triacylglycerol having a chemical structure in which fatty acids are ester-bonded to the three hydroxyl groups of glycerol. Since glycerol is a polyol with three carbon atoms, the lipid is a polyol ester.

According to Experiment 40, it is possible to easily obtain industrially useful chemical components such as a combustible gas, an alcohol, an organic acid, and a ketone by decomposing glycerol with a metal oxide or a metal hydroxide including lead oxide. This result revealed that the polyol ester is also decomposed by water and a metal oxide. The metal oxide has a function of cleaving a carbon-carbon bond even when the hydroxyl group of the polyol in a water-containing state is left as it is or even when the hydroxyl group is esterified.

Triacetin is obtained by acetic acid esterification of three hydroxyl groups of glycerol. According to Experiment 41, it is possible to easily obtain industrially useful chemical components such as a combustible gas, an alcohol, an organic acid, and a ketone by decomposing triacetin with a metal oxide or a metal hydroxide including lead oxide. Further, according to Experiment 42, it is possible to easily obtain industrially useful chemical components such as a combustible gas, an alcohol, an organic acid, a ketone, an alkane, and an alkene by decomposing soybean oil with a metal oxide or a metal hydroxide including lead oxide. Furthermore, according to Experiment 43, it is possible to easily obtain industrially useful chemical components such as a combustible gas, an alcohol, an organic acid, and a ketone by decomposing crude palm oil with a metal oxide or a metal hydroxide including lead oxide. From these results, it can be seen that vegetable oils contain various types of fatty acids, which are bonded in various combinations depending on the type of plant; however, it is possible to decompose glycerol esters with water and a metal oxide or a metal hydroxide to convert them into energy resources and chemical raw materials regardless of such conditions.

According to Experiment 44, it is possible to easily obtain industrially useful chemical components such as a combustible gas, an alcohol, an organic acid, and a ketone by decomposing PKS with a metal oxide or a metal hydroxide including lead oxide.

According to Experiment 45, it is possible to easily obtain industrially useful chemical components such as a combustible gas, an alcohol, an organic acid, and a ketone by decomposing sludge of POME with a metal oxide or a metal hydroxide including lead oxide.

According to Experiment 46, it is possible to easily obtain industrially useful chemical components such as a combustible gas, an alcohol, an organic acid, and a ketone by decomposing an aqueous solution of POME with a metal oxide or a metal hydroxide including lead oxide.

The substances to be decomposed, metal oxides or metal hydroxides, solvents, the presence or absence of acids, bases, and the like, and the types of products according to Experiments 21 to 46 are summarized in Tables 5 to 8.

**[Table 5-1]**

| No | Decomposition target | Metal oxide Metal hydroxide | Solvent | Acid/Base | Product | | |
|---|---|---|---|---|---|---|---|
| | | | | | Hydrogen | Carbon monoxide | Methane |
| Experiment 21 | Starch | Lead oxide | Ultrapure water | | ○ | ○ | |
| Experiment 22 | Cellulose | Lead oxide | Ultrapure water | | ○ | | |
| Experiment 23 | Galactose | Lead oxide | Ultrapure water | | ○ | | |
| Experiment 24 | Mannose | Lead oxide | Ultrapure water | | ○ | ○ | |
| Experiment 25 | Arabinose | Lead oxide | Industrial purified water | | ○ | ○ | |
| Experiment 26 | Paper coffee filter for coffee bean residue | Lead oxide | Ultrapure water | | ○ | ○ | |
| Experiment 27 | Paper coffee filter | Lead oxide | Ultrapure water | Sodium hydroxide | ○ | ○ | |
| Experiment 28 | Fructose | Lead oxide | Ultrapure water | | ○ | ○ | |
| Experiment 29 | Sucrose | Lead oxide | Ultrapure water | | ○ | ○ | |
| Experiment 30 | Maltose monohydrate | Lead oxide | Ultrapure water | | ○ | ○ | |
| Experiment 31 | Lactose monohydrate | Lead oxide | Ultrapure water | | ○ | ○ | |
| Experiment 32 | Cellobiose | Lead oxide | Ultrapure water | | ○ | ○ | |
| Experiment 33 | Citrus pectin | Lead oxide | Ultrapure water | | ○ | ○ | |

**[Table 5-2]**

| No | Product | | | | | | | |
|---|---|---|---|---|---|---|---|---|
| | Acetic acid | Formic acid | Ethylene glycol | Diethylene glycol | Acetone | Methanol | Ethanol | 1-propanol |
| Experiment 21 | ○ | ○ | | | ○ | ○ | ○ | |
| Experiment 22 | ○ | ○ | | | ○ | ○ | ○ | |
| Experiment 23 | ○ | ○ | ○ | ○ | ○ | ○ | ○ | |
| Experiment 24 | ○ | ○ | ○ | | ○ | ○ | ○ | |
| Experiment 25 | ○ | ○ | | | ○ | ○ | | |
| Experiment 26 | ○ | ○ | | | ○ | ○ | ○ | |
| Experiment 27 | ○ | ○ | ○ | ○ | ○ | ○ | ○ | |
| Experiment 28 | ○ | ○ | ○ | ○ | ○ | ○ | ○ | |
| Experiment 29 | ○ | ○ | ○ | | ○ | ○ | ○ | |
| Experiment 30 | ○ | ○ | ○ | ○ | ○ | ○ | ○ | |
| Experiment 31 | ○ | ○ | ○ | ○ | ○ | ○ | ○ | |
| Experiment 32 | ○ | ○ | ○ | | ○ | ○ | ○ | |
| Experiment 33 | ○ | ○ | | | ○ | ○ | ○ | |

**[Table 6-1]**

| No | Decomposition target | Metal oxide Metal hydroxide | Solvent | Acid/Base | Product | | |
|---|---|---|---|---|---|---|---|
| | | | | | Acetoin | Hydroxyacetone | 1-butanol |
| Experiment 21 | Starch | Lead oxide | Ultrapure water | | | | |
| Experiment 22 | Cellulose | Lead oxide | Ultrapure water | | | | |
| Experiment 23 | Galactose | Lead oxide | Ultrapure water | | ○ | | |
| Experiment 24 | Mannose | Lead oxide | Ultrapure water | | | | |
| Experiment 25 | Arabinose | Lead oxide | Industrial purified water | | | | |
| Experiment 26 | Paper coffee filter for coffee bean residue | Lead oxide | Ultrapure water | | | | |
| Experiment 27 | Paper coffee filter | Lead oxide | Ultrapure water | Sodium hydroxide | | | |
| Experiment 28 | Fructose | Lead oxide | Ultrapure water | | | | |
| Experiment 29 | Sucrose | Lead oxide | Ultrapure water | | | | |
| Experiment 30 | Maltose monohydrate | Lead oxide | Ultrapure water | | | | |
| Experiment 31 | Lactose monohydrate | Lead oxide | Ultrapure water | | | | |
| Experiment 32 | Cellobiose | Lead oxide | Ultrapure water | | | | |
| Experiment 33 | Citrus pectin | Lead oxide | Ultrapure water | | | | |

**[Table 6-2]**

| No | Product | | | | | | | |
|---|---|---|---|---|---|---|---|---|
| | 2-hexanol | 3-hexanol | Pentadecane | Octadecane | 8-heptadecene | Hexanoic acid | Heptanoic acid | Octanoic acid |
| Experiment 21 | | | | | | | | |
| Experiment 22 | | | | | | | | |
| Experiment 23 | | | | | | | | |
| Experiment 24 | | | | | | | | |
| Experiment 25 | | | | | | | | |
| Experiment 26 | | | | | | | | |
| Experiment 27 | | | | | | | | |
| Experiment 28 | | | | | | | | |
| Experiment 29 | | | | | | | | |
| Experiment 30 | | | | | | | | |
| Experiment 31 | | | | | | | | |
| Experiment 32 | | | | | | | | |
| Experiment 33 | | | | | | | | |

**[Table 7-1]**

| No | Decomposition target | Metal oxide Metal hydroxide | Solvent | Acid/Base | Product | | |
|---|---|---|---|---|---|---|---|
| | | | | | Hydrogen | Carbon monoxide | Methane |
| Experiment 34 | Dealkalized lignin | Lead oxide | Ultrapure water | | ○ | ○ | |
| Experiment 35 | Cypress powder | Lead oxide | Ultrapure water | | ○ | ○ | |
| Experiment 36 | Xylan | Lead oxide | Ultrapure water | | ○ | ○ | |
| Experiment 37 | Xylose | Lead oxide | Ultrapure water | | ○ | ○ | |
| Experiment 38 | Domestic sludge | Lead oxide | Industrial purified water | | ○ | ○ | |
| Experiment 39 | Papermaking sludge | Lead oxide | Industrial purified water | | ○ | ○ | |
| Experiment 40 | Glycerol | Lead oxide | Ultrapure water | | ○ | ○ | |
| Experiment 41 | Triacetin | Lead oxide | Ultrapure water | | ○ | ○ | |
| Experiment 42 | Soybean oil | Lead oxide | Industrial purified water | | ○ | ○ | ○ |
| Experiment 43 | Crude palm oil | Lead oxide | Ultrapure water | Sulfuric acid | ○ | ○ | ○ |
| Experiment 44 | Palm kernel shell | Lead oxide | Ultrapure water | Sulfuric acid | ○ | ○ | ○ |
| Experiment 45 | Sludge in palm oil mill effluent | Lead oxide | Ultrapure water | Sulfuric acid | ○ | ○ | |
| Experiment 46 | Aqueous solution of palm oil mill effluent | Lead oxide | × | Sulfuric acid | ○ | ○ | ○ |

**[Table 7-2]**

| No | Product | | | | | | | |
|---|---|---|---|---|---|---|---|---|
| | Acetic acid | Formic acid | Ethylene glycol | Diethylene alycol | Acetone | Methanol | Ethanol | 1-propanol |
| Experiment 34 | ○ | | | | ○ | ○ | ○ | |
| Experiment 35 | ○ | | | | ○ | ○ | ○ | |
| Experiment 36 | ○ | ○ | ○ | ○ | ○ | ○ | ○ | |
| Experiment 37 | ○ | ○ | | | ○ | ○ | ○ | |
| Experiment 38 | ○ | ○ | ○ | ○ | ○ | ○ | ○ | |
| Experiment 39 | ○ | ○ | ○ | ○ | ○ | ○ | ○ | |
| Experiment 40 | ○ | ○ | ○ | | ○ | ○ | ○ | |
| Experiment 41 | ○ | ○ | ○ | | ○ | ○ | ○ | |
| Experiment 42 | ○ | ○ | ○ | | ○ | ○ | ○ | |
| Experiment 43 | ○ | ○ | ○ | ○ | ○ | ○ | ○ | ○ |
| Experiment 44 | ○ | ○ | ○ | ○ | ○ | ○ | ○ | |
| Experiment 45 | ○ | ○ | ○ | ○ | ○ | ○ | ○ | |
| Experiment 46 | ○ | ○ | ○ | ○ | ○ | ○ | ○ | ○ |

**[Table 8-1]**

| No | Decomposition target | Metal oxide Metal hydroxide | Solvent | Acid/Base | Product | | |
|---|---|---|---|---|---|---|---|
| | | | | | _{A}cetoin | Hydroxyacetone | 1-butanol |
| Experiment 34 | Dealkalized lignin | Lead oxide | Ultrapure water | | | | |
| Experiment 35 | Cypress powder | Lead oxide | Ultrapure water | | | | |
| Experiment 36 | Xylan | Lead oxide | Ultrapure water | | | | |
| Experiment 37 | Xylose | Lead oxide | Ultrapure water | | | | |
| Experiment 38 | Domestic sludge | Lead oxide | Industrial purified water | | | | |
| Experiment 39 | Papermaking sludge | Lead oxide | Industrial purified water | | | | |
| Experiment 40 | Glycerol | Lead oxide | Ultrapure water | | | | |
| Experiment 41 | Triacetin | Lead oxide | Ultrapure water | | | | |
| Experiment 42 | Soybean oil | Lead oxide | Industrial purified water | | | ○ | ○ |
| Experiment 43 | Crude palm oil | Lead oxide | Ultrapure water | Sulfuric acid | | | |
| Experiment 44 | Palm kernel shell | Lead oxide | Ultrapure water | Sulfuric acid | | | |
| Experiment 45 | Sludge in palm oil mill effluent | Lead oxide | Ultrapure water | Sulfuric acid | | | |
| Experiment 46 | Aqueous solution of palm oil mill effluent | Lead oxide | × | Sulfuric acid | | | |

**[Table 8-2]**

| No | Product | | | | | | | |
|---|---|---|---|---|---|---|---|---|
| | 2-hexanol | 3-hexanol | Pentadecane | Octadecane | 8-heptadecene | Hexanoic acid | Heptanoic acid | Octanoic acid |
| Experiment 34 | | | | | | | | |
| Experiment 35 | | | | | | | | |
| Experiment 36 | | | | | | | | |
| Experiment 37 | | | | | | | | |
| Experiment 38 | | | | | | | | |
| Experiment 39 | | | | | | | | |
| Experiment 40 | | | | | | | | |
| Experiment 41 | | | | | | | | |
| Experiment 42 | ○ | ○ | ○ | ○ | ○ | ○ | ○ | ○ |
| Experiment 43 | | | | | | | | |
| Experiment 44 | | | | | | | | |
| Experiment 45 | | | | | | | | |
| Experiment 46 | | | | | | | | |

Biologically derived polyols are a collection group of glucose and a wide variety of other monosaccharides produced by photosynthesis of plants, disaccharides and oligosaccharides that are combinations of monosaccharides, and sugar skeletons obtained by polymerizing these. While the basic unit of the sugar skeleton is an extremely simple chemical structure of [HCOH], since there are various combinations in conformation (configuration), configuration (conformation), and bonding positions between monosaccharides, biologically derived polyols are very diverse. Enzymes are responsible for promoting an ordered chemical reaction in a cell based on substrate specificity, and polyols contained in waste have a chemical structure that is difficult to be decomposed by an enzyme. Therefore, it is not efficient to decompose various biologically derived polyols contained in the waste by an enzyme except for a specific reaction system. Further, the biologically derived polyol esters are triglycerides. There are various types of triglycerides, and there are also a wide variety of lipases which are enzymes decomposing these triglycerides. For all the types of triglycerides contained in the waste, it is not desirable in terms of cost to prepare corresponding lipases to treat the waste. In other words, the substrate specificity of each lipase is rather disadvantageous in terms of waste treatment. Further, similarly to the polyol, the polyol ester remaining as a waste is not easily decomposed by an enzyme. According to Experiments 1 to 46, an oxidation action or reduction action of water caused in water by metal oxides and metal hydroxides, which have standard oxidation-reduction potentials different from that of water, and a hydrolysis action of organic substances in an acidic environment or an alkaline environment near the surface have an advantage of not having structure selectivity represented by the substrate specificity like an enzyme to a wide variety of polyols and polyol esters.

### [Experiment 47]

In Experiment 47, 3.0195 g (0.070 mol) in weight of polyvinyl alcohol (PVA, 43 g/mol, manufactured by FUJIFILM Wako Pure Chemical Corporation), 27.0079 g of lead oxide (PbO₂, manufactured by KANTO CHEMICAL CO., INC.), and 50 mL of industrial purified water were placed in a pressure-resistant container (MMJ-100 manufactured by OMLABO TECH CO., LTD., capacity 120 mL), and the container was sealed. At this point, the concentration of PVA is 0.14 mol/L. In this state, a reaction was carried out at 200 °C for 1.5 hours while stirring at a rotation speed of 400 rpm. As a result, from the gas phase, 0.0093 mol (13.3 mol%) of hydrogen and 0.00025 mol (0.36 mol%) of carbon monoxide were obtained, and from the liquid phase, as aqueous solutions, 0.026 mol/L (18.4 mol%) of acetone, 0.0049 mol/L (3.5 mol%) of methanol, 0.00054 mol/L (0.39 mol%) of ethanol, 0.0075 mol/L (5.3 mol%) of acetic acid, 0.0063 mol/L (4.5 mol%) of formic acid, 0.00046 mol/L (0.33 mol%) of ethylene glycol, and 0.0012 mol/L (0.84 mol%) of diethylene glycol were obtained.

Polyvinyl alcohol (PVA) serves as an artificial polyol and is widely used as a thickener, an adhesive, an emulsifier and the like in various industrial processes. Therefore, it is often contained in waste liquid, waste, and sludge. According to Experiment 47, it is possible to easily obtain industrially useful chemical components such as a combustible gas, an alcohol, an organic acid, and a ketone by decomposing PVA with a metal oxide or a metal hydroxide including lead oxide.

### [Experiment 48]

In Experiment 48, 1.0097 g in weight of a ground product of PET bottles (5 mm square on average, provided by Otsuka Pharmaceutical Co., Ltd., washed), 6.5113 g of powdered lead oxide (IV) (PbO₂, manufactured by KANTO CHEMICAL CO., INC., purity 97.0%), 50 mL of ultrapure water (18.2 MQ-cm, manufactured by ELGA), and a rugby ball type stirrer (Teflon coated) having a length of 25 mm were placed in a pressure-resistant container (NR0218 manufactured by Flonchemical Co., Ltd., capacity 100 mL), and the container was sealed. Then, the pressure-resistant container was heated to 190 °C using a temperature controller, and then the mixture was allowed to react for 1 hour while the stirrer was rotated at 20 rpm, in a state where the temperature was maintained at 190 °C (±5 °C). Thereafter, the pressure-resistant container was allowed to cool naturally to room temperature, and suction filtration was then performed to measure components contained in the filtrate. Note that the pH of the filtrate was between 6 and 7. Further, a large quantity of the ground product of PET bottles (hereinafter, also referred to as PET ground product) remained on the filter together with the lead oxide powder. Fig. 3 is a chromatogram showing measurement results of GCMS of the filtrate. As can be seen from this chromatogram, as components contained in the filtrate, ethylene glycol (82%) was the predominant component, and diethylene glycol (9%) and methanol (9%) were only present in small amounts. Further, formic acid was also observed in a small amount.

### [Experiment 49]

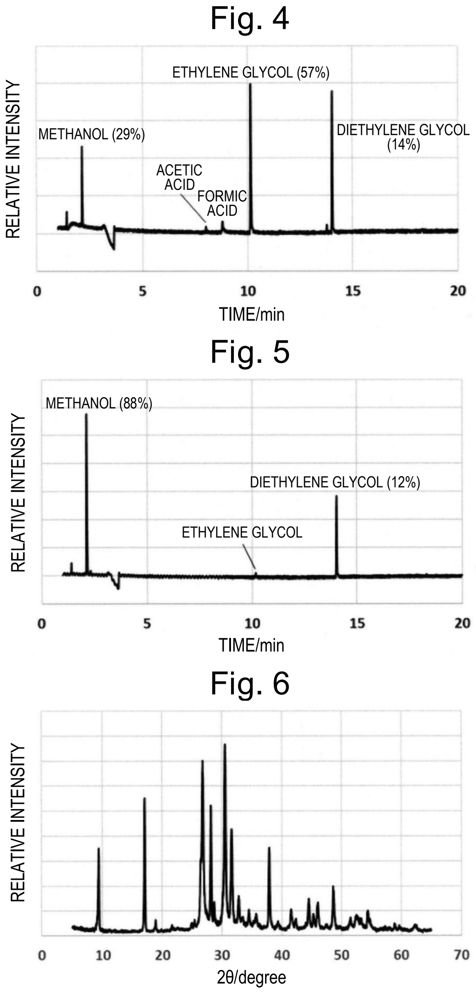

Experiment 49 was performed in the same procedure as in Experiment 48 except that the amount of the ground product of used PET bottles added was changed to 1.0160 g, the amount of powdered lead oxide (IV) added was changed to 6.5110 g, and the reaction time was changed to 3 hours. The same materials, containers, and the like as those used in Experiment 48 were used. After the reaction, the pressure-resistant container was allowed to cool naturally to room temperature, and then suction filtration was performed to measure the components contained in the filtrate. The pH of the filtrate was between 6 and 7. A small amount of PET ground product remained on the filter together with the lead oxide powder. The chromatogram obtained by GCMS is shown in Fig. 4. As can be seen from this chromatogram, as the component contained in the filtrate, ethylene glycol (57%) was the predominant component. Further, as compared with the results of Experiment 48, a proportion of ethylene glycol was decreased, and the proportions of diethylene glycol (14%) and methanol (29%) were increased instead. In particular, the proportion of methanol was about three times the result of Experiment 48 (9%). Acetic acid and formic acid were also observed.

### [Experiment 50]

An experiment was performed in the same procedure as in Experiment 48 except that the amount of the ground product of used PET bottles added was changed to 1.0155 g, the amount of powdered lead oxide (IV) added was changed to 6.5139 g, and the reaction time was changed to 6 hours. The same materials, containers, and the like as those used in Experiment 48 were used. After the reaction, the pressure-resistant container was allowed to cool naturally to room temperature, and then suction filtration was performed to measure the components contained in the filtrate. The pH of the filtrate was between 6 and 7. Further, no lead oxide powder or PET ground product were observed on the filter. From this, it was found that when the reaction temperature was 190 °C, depolymerization of PET was completed within 6 hours. The chromatogram obtained by GCMS is shown in Fig. 5. From this chromatogram, it was found that the main component of the components contained in the filtrate was methanol (88%), and ethylene glycol was below a detection limit. From this, it was considered that almost all ethylene glycol was converted into methanol. On the other hand, diethylene glycol (12%) remained without being converted to ethanol.

In order to confirm that terephthalic acid was attached to the surface of the lead oxide powder on the filter, the lead oxide powder was placed again in a pressure-resistant container, to which 50 mL of ultrapure water (18.2 MΩ·cm, manufactured by ELGA) and 0.44 g of sodium hydroxide were added. Then, a rugby ball type stirrer (Teflon-coated) having a length of 25 mm was placed in the pressure-resistant container, which was then sealed, and the mixture was allowed to react at 190 °C (±5 °C) for 4 hours.

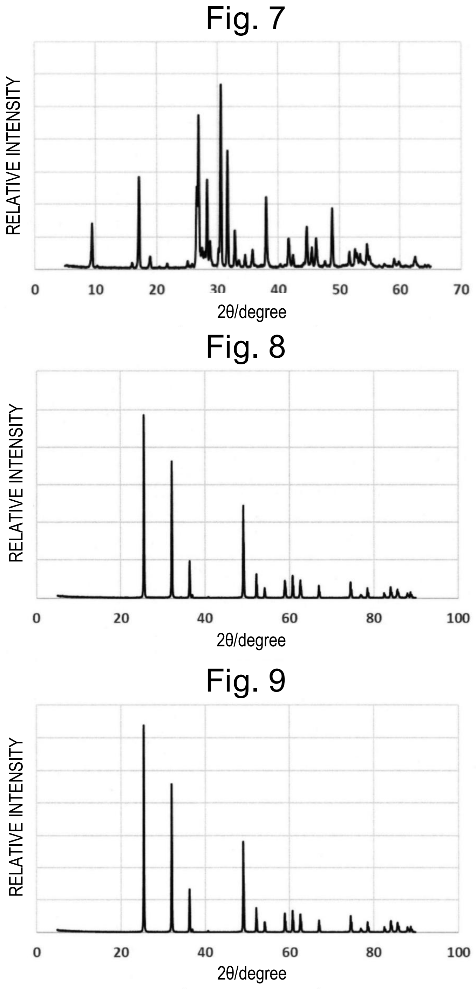

Thereafter, the pressure-resistant container was allowed to cool naturally to room temperature, and then suction filtration was performed. The lead oxide powder remained on the filter. Further, the filtrate was placed in a dryer at 90 °C, and water was completely removed over 8 hours. As a result, 0.95 g of a slightly cream-colored white powder was obtained. The XRD spectrum obtained from the X-ray diffraction data of this powder is shown in Fig. 6. Further, the XRD spectrum of disodium terephthalate (Tokyo Chemical Industry Co., Ltd.), a commercially available experimental reagent, is shown in Fig. 7.

From Figs. 6 and 7, it was found that the substance attached to the lead oxide powder was disodium terephthalate, and the yield of disodium terephthalate derived from used PET was 85%.

Further, in order to confirm that the lead oxide powder remaining on the filter was the same as the original lead oxide (PbO₂), X-ray diffraction measurement was performed on this powder and an unused lead oxide powder. As a result, the XRD spectra of lead oxide remaining on the filter and unused lead oxide were as shown in Figs. 8 and 9.

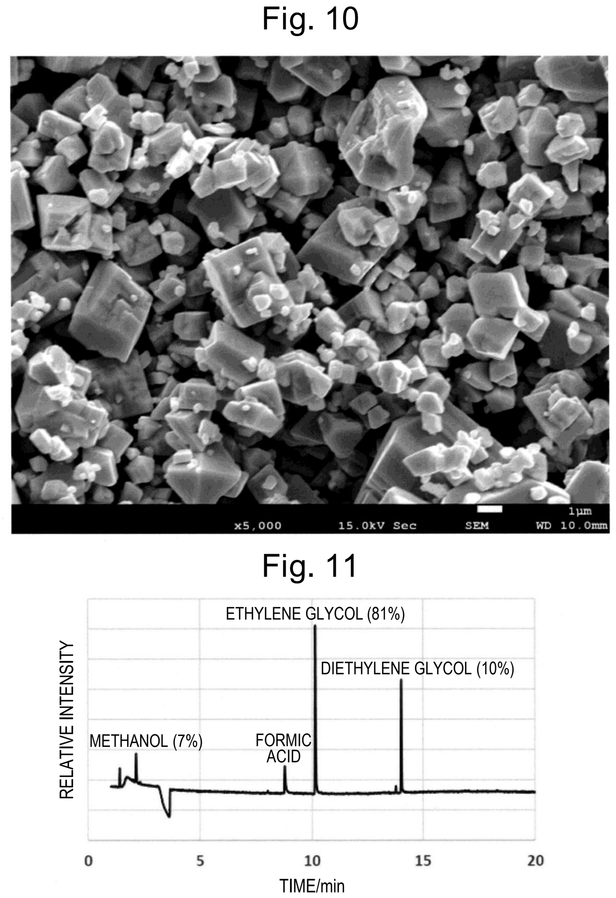

A comparison between Fig. 8 and Fig. 9 showed that lead oxide did not change before and after a depolymerization reaction of PET, and worked as a solid-liquid interface catalyst. Further, Fig. 10 shows an electron microscope (SEM) observation image of the unused lead oxide powder. From Fig. 10, it can be seen that the lead oxide powder used in this experiment is a powder having a maximum length of about 0.1 to 3.0 µm.

### [Experiment 51]

An experiment was performed in the same procedure as in Experiments 48 to 50 except that the temperature at which the PET ground product was reacted with water using lead oxide as a solid-liquid interface catalyst in the reaction container was changed to 200 °C (±5 °C). In this experiment, when the reaction time was set to 1 hour, the amount of used PET bottles added was 1.0037 g, the amount of powdered lead oxide added was 6.5046 g, when the reaction time was set to 3 hours, the amount of used PET bottles added was 1.0027 g, the amount of powdered lead oxide added was 6.5103 g, when the reaction time was set to 6 hours, the amount of used PET bottles added was 1.0026 g, and the amount of powdered lead oxide added was 6.5123 g. Further, in each case, the amount of ultrapure water added was 50 mL. As a result of the experiment, chromatograms that are the measurement results of GCMS of the components contained in the obtained filtrate are shown in Figs. 11 to 13. Figs. 11, 12, and 13 show the results when the reaction time between the PET ground product and water was set to 1 hour, 3 hours, and 6 hours, respectively.

As can be seen from the comparison between Figs. 11 to 13 and Figs. 3 to 5, when the reaction temperature was set to 200 °C, the same results were obtained as when the reaction temperature was set to 190 °C.

### [Comparative Experiment]

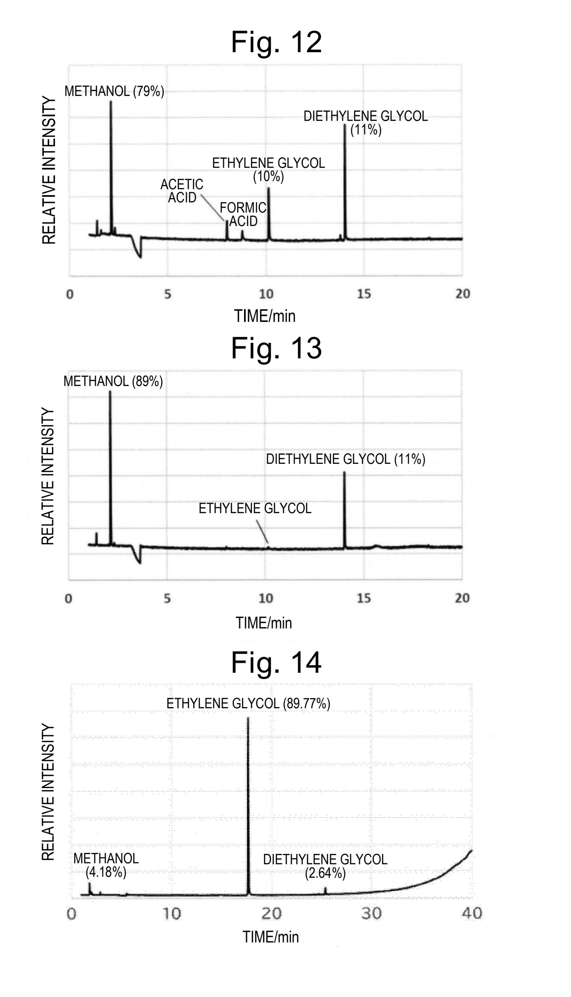

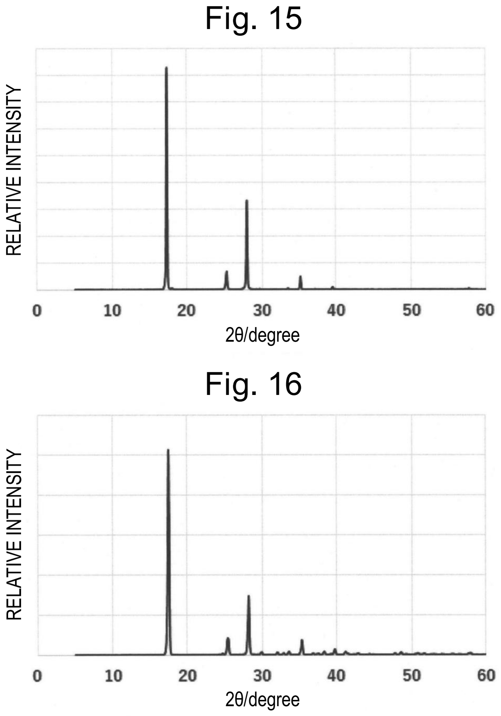

As a comparative experiment, an experiment similar to Experiments 48 to 50 was performed without using powdered lead oxide. In other words, 1.0134 g of the ground product of the collected used PET bottles (5 mm square on average, provided by Otsuka Pharmaceutical Co., Ltd., washed) was placed in a pressure-resistant container (NR0218 manufactured by Flonchemical Co., Ltd., capacity 100 mL). 50 mL of ultrapure water (18.2 MΩ·cm, manufactured by ELGA) was added to the container. Then, a rugby ball type stirrer (Teflon coated) having a length of 25 mm was put in the pressure-resistant container and the container was sealed. The pressure-resistant container was heated to 200 °C with a temperature controller, and then the mixture was allowed to react for 72 hours while the stirrer was rotated at 20 rpm, in a state where the temperature was maintained at 200 °C (±5 °C). Thereafter, the reaction container was allowed to cool naturally to room temperature, and then suction filtration was performed to measure components contained in the filtrate. The pH of the filtrate was between 4 and 5. A white powder remained on the filter. For the measurement of the components contained in the filtrate, the same GCMS as in Experiments 48 to 50 was used. The measurement conditions of the GCMS conform to the measurement conditions of Experiments 48 to 50, but in order to carefully examine whether or not conversion of ethylene glycol to methanol has occurred, a temperature rising rate of the column was set to 5 °C/min, which is slower than that in Experiment 48. Fig. 14 is a chromatogram showing the measurement results of the filtrate. From Fig. 14, it was found that ethylene glycol can be obtained from PET by prolonging the reaction time even without lead oxide powder. On the other hand, it has been confirmed that without the lead oxide powder, ethylene glycol obtained from PET is hardly converted into methanol even when the reaction time is lengthened. Further, in order to confirm that the white powder remaining on the filter was terephthalic acid, X-ray diffraction measurement of the white powder was performed. The resulting XRD spectrum is shown in Fig. 15, and the XRD spectrum of the standard substance is shown in Fig. 16. As can be seen from Figs. 15 and 16, when PET was depolymerized only with water, terephthalic acid could be recovered, but methanol was hardly obtained. This result was consistent with the result in Fig. 14.

Note that, it is reported in Patent Literature 1 that PET is hydrolyzed using water as a medium to obtain terephthalic acid and ethylene glycol under a temperature condition of 200 °C to 300 °C. However, it is said that the temperature condition in the case of using heat energy (exhaust heat energy) discharged from a chemical factory, a food factory, a steel mill, a cement factory, and the like is 200 °C at most, and under the temperature condition of 200 °C to 300 °C, it is not possible to cope with only using the exhaust heat energy, and an additional heat source is required. Regarding this, in the present example, PET can be sufficiently hydrolyzed to obtain terephthalic acid and ethylene glycol even under a temperature condition of 190 °C to 200 °C, and furthermore, by prolonging the reaction time, it is possible to convert ethylene glycol into methanol. In other words, in the present example, since exhaust heat energy of a chemical factory, a food factory, or the like can be used without adding a heat source, it can be said that the method can contribute to reduction of carbon dioxide emission.

### [Experiment 52]

In Experiment 52, 10.35725 g (0.054 mol) in weight of polyester resin (PET) flakes (approximately 192 g/mol, a crush product of collected plastic bottles, 5 mm square on average), 90.2639 g of lead oxide (PbO₂, manufactured by KANTO CHEMICAL CO., INC.) and 300 mL of industrial purified water were placed in a pressure-resistant container (TEM-D1000M manufactured by Taiatsu Techno Corporation, capacity 1550 mL), and the container was sealed. At this point, the concentration of PET is 0.18 mol/L assuming that all the PET flakes are dissolved. In this state, a reaction was carried out at 200 °C for 8 hours while stirring at a rotation speed of 200 rpm. As a result, from the gas phase, 0.013 mol (23.7 mol%) of hydrogen, 0.00041 mol/L (0.76 mol%) of carbon monoxide, and 0.00021 mol (0.38mol%) of methane were obtained, and from the liquid phase, as an aqueous solution, 0.059 mol/L (32.5 mol%) of methanol and trace amount of diethylene glycol were obtained.

### [Experiment 53]

In Experiment 53, 3.0165 g (0.486 mol) in weight of ethylene glycol (EG, 62 g/mol, manufactured by FUJIFILM Wako Pure Chemical Corporation), 27.5042 g of lead oxide (PbO₂, manufactured by KANTO CHEMICAL CO., INC.), and 50 mL of industrial purified water were placed in a pressure-resistant container (MMJ-100 manufactured by OMLABO TECH CO., LTD., capacity 120 mL), and the container was sealed. At this point, the concentration of EG is 0.97 mol/L. In this state, a reaction was carried out at 200 °C for 30 minutes while stirring at a rotation speed of 400 rpm. As a result, from the gas phase, 0.36 mol (36 mol%) of hydrogen and 0.0021 mol/L (0.2 mol%) of carbon monoxide were obtained, and from the liquid phase, as aqueous solutions, 0.79 mol/L (80.9 mol%) of methanol and 0.029 mol/L (3.0 mol%) of acetic acid and 0.069 mol/L (7.1 mol%) of ethylene glycol were obtained.

According to Experiments 48 to 51, it is possible to easily obtain EG and the like by decomposing a polyester resin (PET) with a metal oxide or a metal hydroxide including lead oxide. According to Experiment 52, it is possible to easily obtain industrially useful chemical components such as a combustible gas, other alcohols, and organic acids.

According to Experiment 53, it is possible to easily obtain industrially useful chemical components such as a combustible gas, an alcohol, and an organic acid by decomposing EG with a metal oxide or a metal hydroxide including lead oxide.

The substances to be decomposed, metal oxides or metal hydroxides, solvents, the presence or absence of acids, bases, and the like, and the types of products according to Experiments 47 to 53 are summarized in Table 9.

**[Table 9-1]**

| No | Decomposition target | Metal oxide/hydroxide | Solvent | Acid/Base | Product | | |
|---|---|---|---|---|---|---|---|
| | | | | | Hydrogen | Carbon monoxide | Methane |
| Experiment 47 | Polyvinyl alcohol | Lead oxide | Industrial purified water | | ○ | ○ | |
| Experiment 48 | PET flake | Lead oxide | Ultrapure water | | | | |
| Experiment 49 | PET flake | Lead oxide | Ultrapure water | | | | |
| Experiment 50 | PET flake | Lead oxide | Ultrapure water | | | | |
| Experiment 51 | PET flake | Lead oxide | Ultrapure water | | | | |
| Comparative experiment | PET flake | × | Ultrapure water | | | | |
| Experiment 52 | PET flake | Lead oxide | Industrial purified water | | ○ | ○ | ○ |
| Experiment 53 | Ethylene glycol | Lead oxide | Industrial purified water | | ○ | ○ | |

**[Table 9-2]**

| No | Product | | | | | | | | |
|---|---|---|---|---|---|---|---|---|---|
| | Acetic acid | Formic acid | Ethylene glycol | Diethylene glycol | Acetone | Methanol | Ethanol | Disodium terephthalate | Terephthalic acid |
| Experiment 47 | ○ | ○ | ○ | ○ | ○ | ○ | ○ | | |
| Experiment 48 | | ○ | ○ | | | | | | |
| Experiment 49 | ○ | ○ | ○ | ○ ○ | | ○ ○ | | | |
| Experiment 50 | | | | ○ | | ○ | | ○ | |
| Experiment 51 | ○ | ○ | ○ | ○ | | ○ | | ○ | |
| Comparative experiment | | | ○ | | | | | | ○ |
| Experiment 52 | | | | ○ | | ○ | | | |
| Experiment 53 | ○ | | ○ | | | ○ | | | |

80% or more of the whole of food waste including leftovers, industrial waste discharged from a food plant, a sewage treatment plant, or a paper mill, sludge, and the like is moisture. Further, the organic substances contained in these wastes are derived from animals and plants, and have a very high affinity for water. Therefore, a huge amount of fossil fuels has been conventionally consumed to dry and incinerate these wastes. In order to utilize these wastes as resources, it was necessary to efficiently dispose of or utilize this 80% water content. By applying Experiments 1 to 49, one can draw a clear vision of utilizing water, which makes up 80% of the total weight of the waste, to extract useful chemicals such as hydrogen, carbon monoxide, methanol and ethanol. Further, in Experiments 1 to 49, it is not necessary to use an expensive chemical substance, a chemical, a complicated device, or the like. Therefore, Experiments 1 to 49 will be rapidly socially implemented using inexpensive and mass-produced materials.

The oxidation action or reduction action of water caused in water by metal oxides and metal hydroxides, which have standard oxidation-reduction potentials different from that of water, and the hydrolysis action of organic substances in an acidic environment or an alkaline environment near the surface occur only near the solid-liquid interface regardless of the pH of the bulk aqueous phase and the presence of the inorganic salt. Therefore, as shown in Experiments 10, 11, 13, 14, 27, and 43 to 46, an acid (sulfuric acid, phosphoric acid, carbonic acid, and the like), an alkali or a salt (sodium hydroxide, potassium hydroxide, calcium hydroxide, calcium oxide, sodium carbonate, sodium hydrogen carbonate, and the like), or a water-soluble oxide (calcium hypochlorite, sodium percarbonate, and the like) that has been conventionally used for hydrolyzing a polymer compound may be contained. By containing these acids, alkalis, and inorganic salts, a high molecular weight polyol or polyol ester can be hydrolyzed in the bulk aqueous phase to some extent in advance to be in a low molecular weight state, so that efficiency of hydrolysis can be improved, or a production ratio of products can be changed. Further, for the same reason, it is also possible to make a high molecular weight polyol or polyol ester have a low molecular weight in advance in the bulk aqueous phase by allowing a water-soluble oxide (calcium hypochlorite, sodium percarbonate, and the like) to coexist with a metal oxide. Calcium hypochlorite (bleaching powder) and sodium percarbonate are often used for cleaning in general households, and have the advantages of high safety and low price.

### [Modes]

A person skilled in the art can understand that the previously described illustrative embodiment is a specific example of the following modes of the present invention.

(Clause 1) A method for decomposing a polyol or a polyol ester according to one mode of the present invention includes
bringing, in predetermined reaction water, a substance to be decomposed containing at least one of a polyol and a polyol ester into contact with a solid of a metal oxide or a metal hydroxide having a standard oxidation-reduction potential different from that of water.

(Clause 2) A method for decomposing a polyol or a polyol ester according to Clause 2 is the method for decomposing a polyol or a polyol ester according to Clause 1, in which the substance to be decomposed is heated to 100 °C or higher in a state of being brought into contact with the solid in the predetermined reaction water.

(Clause 3) A method for decomposing a polyol or a polyol ester according to Clause 3 is the method for decomposing a polyol or a polyol ester according to Clause 1 or 2, in which the substance to be decomposed includes any one or more of glucose, starch, cellulose, galactose, mannose, arabinose, fructose, sucrose, maltose, lactose, cellobiose, pectin, lignin, xylan, xylose, glycerol, triacetin, glyceride, polyvinyl alcohol, polyester, and ethylene glycol.

(Clause 4) A method for decomposing a polyol or a polyol ester according to Clause 4 is the method for decomposing a polyol or a polyol ester according to any one of Clauses 1 to 3, in which the solid includes any one or more of alumina (Al₂O₃), silica (SiO₂), zeolite (Al₂O₃·SiO₂), titania (TiO₂), vanadium pentoxide (V₂O₅), manganese dioxide (MnO₂), iron hydroxide (FeO(OH)), zinc oxide (ZnO), germanium oxide (GeO₂), tin oxide (SnO₂), lead oxide (PbO₂), nickel oxide (NiO₂, Ni₃O₄), and nickel hydroxide (Ni(OH)₃).

In the method for decomposing a polyol or a polyol ester according to Clauses 1 to 4, a solid of a metal oxide or a metal hydroxide having a standard oxidation-reduction potential different from that of water is added to predetermined reaction water. At this time, a reductant of a system having a lower standard oxidation-reduction potential is more likely to release electrons, and an oxidant of a system having a higher standard oxidation-reduction potential is more likely to accept electrons. Therefore, it is considered that on the surface of the solid of the metal oxide or the metal hydroxide having a standard oxidation-reduction potential higher than that of water, water molecules (H₂O) are spontaneously oxidized to generate protons (H⁺), electrons (e⁻), and oxygen (O₂), and the solid surface (solid-liquid interface) of the metal oxide or the metal hydroxide becomes a strong acidic environment. Further, it is considered that on the surface of the solid of the metal oxide or the metal hydroxide having a standard oxidation-reduction potential lower than that of water, water molecules (H₂O) are spontaneously reduced to generate hydrogen (H₂) and hydroxide ions (OH⁻), and the solid surface (solid-liquid interface) of the metal oxide or the metal hydroxide becomes a strong alkaline environment.

However, only an area near the solid-liquid interface becomes a strong acidic environment or alkaline environment, and it does not mean that the entire water (bulk) becomes an acidic environment or alkaline environment. Therefore, in the method for decomposing a polyol or a polyol ester according to Clauses 1 to 4, a substance to be decomposed containing at least one of a polyol and a polyol ester is brought into contact with the surface of a solid of a metal oxide or a metal hydroxide in predetermined reaction water. As a result, the substance to be decomposed is hydrolyzed.

Therefore, according to the method for decomposing a polyol or a polyol ester according to Clauses 1 to 4, by bringing, in predetermined reaction water, a substance to be decomposed containing at least one of a polyol and a polyol ester into contact with a solid of a metal oxide or a metal hydroxide having a standard oxidation-reduction potential different from that of water, the polyol or the polyol ester can be more easily decomposed. In particular, according to the method for decomposing a polyol or a polyol ester according to Clause 2, by heating the substance to be decomposed to 100 °C or higher in a state of being brought into contact with the solid in the predetermined reaction water, the rate of hydrolysis can be increased.

(Clause 5) A method for decomposing a polyol or a polyol ester according to Clause 5 is the method for decomposing a polyol or a polyol ester according to any one of Clauses 1 to 4, in which the substance to be decomposed is in the form of small pieces.

(Clause 6) A method for decomposing a polyol or a polyol ester according to Clause 6 is the method for decomposing a polyol or a polyol ester according to any one of Clauses 1 to 5, in which the solid is in the form of particles or powder.

According to the method for decomposing a polyol or a polyol ester according to Clause 5 or 6, the contact area between the substance to be decomposed and the solid of the metal oxide and the metal hydroxide can be increased to increase the rate of the hydrolysis reaction.

(Clause 7) A method for decomposing a polyol or a polyol ester according to Clause 7 is the method for decomposing a polyol or a polyol ester according to any one of Clauses 1 to 6, in which the reaction water contains any one of an acid, an alkali, a salt, and a water-soluble oxide.

According to the method for decomposing a polyol or a polyol ester according to Clause 7, a substance to be decomposed containing a high molecular weight polyol or polyol ester can be hydrolyzed in advance to some extent in a bulk aqueous phase to be in a low molecular weight state, so that the efficiency of hydrolysis can be improved, and the production ratio of products can be changed.

(Clause 8) A production method according to Clause 8 uses the method for decomposing a polyol or a polyol ester according to any one of Clauses 1 to 7 to produce any one of a combustible gas, an alcohol, an organic acid, a ketone, an aldehyde, a lactone, an alkane, and an alkene.

According to the production method according to Clause 8, an industrially useful chemical component such as a combustible gas, an alcohol, an organic acid, a ketone, an aldehyde, a lactone, or an alkene can be easily obtained by decomposing a polyol or a polyol ester.

## Claims

1. A method for decomposing a polyol or a polyol ester, the method comprising bringing, in predetermined reaction water, a substance to be decomposed containing at least one of a polyol and a polyol ester into contact with a solid of a metal oxide or a metal hydroxide having a standard oxidation-reduction potential different from that of water,
wherein the substance to be decomposed is heated to a temperature range between 100 °C and 250 °C in a state of being brought into contact with the solid in the predetermined reaction water.

2. The method for decomposing a polyol or a polyol ester according to claim 1, wherein the substance to be decomposed includes any one or more of glucose, starch, cellulose, galactose, mannose, arabinose, fructose, sucrose, maltose, lactose, cellobiose, pectin, lignin, xylan, xylose, glycerol, triacetin, glyceride, polyvinyl alcohol, polyester, and ethylene glycol.

3. The method for decomposing a polyol or a polyol ester according to claim 1 or 2, wherein the solid includes any one or more of alumina (Al₂O₃), silica (SiO₂), zeolite (Al₂O₃·SiO₂), titania (TiO₂), vanadium pentoxide (V₂O₅), manganese dioxide (MnO₂), iron hydroxide (FeO(OH)), zinc oxide (ZnO), germanium oxide (GeO₂), tin oxide (SnO₂), lead oxide (PbO₂), nickel oxide (NiO₂, Ni₃O₄), and nickel hydroxide (Ni(OH)₃).

4. The method for decomposing a polyol or a polyol ester according to any one of claims 1 to 3, wherein the substance to be decomposed is in a form of small pieces.

5. The method for decomposing a polyol or a polyol ester according to any one of claims 1 to 4, wherein the solid is in a form of particles or powder.

6. The method for decomposing a polyol or a polyol ester according to any one of claims 1 to 5, wherein the predetermined reaction water contains any of an acid, an alkali, a salt, and a water-soluble oxide.

7. A production method for producing any one of a combustible gas, an alcohol, an organic acid, a ketone, an aldehyde, a lactone, an alkane, and an alkene by using the method for decomposing a polyol or a polyol ester according to any one of claims 1 to 6.
